# EUROPEAN PATENT APPLICATION

(11) **EP 2 530 143 A1**
(43) Date of publication of application: **05.12.2012**
(21) Application number: 11736658.3
(22) Date of filing: 24.01.2011
(51) Int. Cl.: C12N 5/00, C12N 5/07, C12N 5/0783, C07K 16/28

(54) **METHOD FOR THE IDENTIFICATION AND PURIFICATION OF HUMAN NATURALLY OCCURRING REGULATORY T CELLS (NTREGS)**

(30) Priority: 26.01.2010 ES 201030096
(71) Applicant: Universidade De Santiago De Compostela, 15782 Santiago de Compostela (ES)
(72) Inventor: NOGUEIRA ÁLVAREZ, Montserrat, 15782 Santiago de Compostela (ES); SALGADO CASTRO, Francisco Javier, 15782 Santiago de Compostela (ES); ARIAS CRESPO, Pilar, 15782 Santiago de Compostela (ES); PÉREZ DÍAZ, Amparo, 15782 Santiago de Compostela (ES); MARTÍNEZ VILLANUEVA, Nora, 15782 Santiago de Compostela (ES)
(74) Representative: Pons Ariño, Angel
(86) International application number: PCT/ES2011/070037
(87) International publication number: WO 2011/092359

(57) **Abstract**

Method for the isolation and purification of nTreg cells from a biological sample, preferably from an individual having an inflammatory or autoimmune disease. Furthermore, the invention additionally relates to a kit for the isolation or purification of nTreg cells from a biological sample.

## Description

The present invention relates to the field of immunology. Specifically, the invention relates to a method and a kit for the isolation or purification of nTreg cells from a biological sample, preferably from an individual having an inflammatory or autoimmune disease.

### STATE OF THE PRIOR ART

Naturally occurring regulatory T cells CD4+CD25+, also referred to as nTreg cells, represent 5-10% of T CD4+ lymphocytes in peripheral blood. Their function is to inhibit the activation and effector functions (proliferation, cytokine and antibody production, etc.) of other cells of the immune system, the so - called "effector cells", which are those responsible for the immunological response (for example, other T lymphocytes, but also B lymphocytes, NK and NKT cells, antigen - presenting cells, etc.).

Initially it was thought that nTreg cells controlled exclusively the responses of effector cells to self antigens (autoantigens), and that for this reason elimination of this specialised lymphocyte population gave rise to the development of systemic autoimmune diseases. However, it soon became known that they also suppressed responses to foreign antigens (alloantigens) or tumour antigens, in this way controlling the triggering of excessive immune responses (in the case of viral or bacterial infections, for example) or preventing the generation of immune responses (such as, for example, in the case of cancer or AIDS).

There is, therefore, a growing interest in researching the role of nTreg cells in the context of certain diseases (infections, autoimmune diseases, cancer or AIDS) as well as in transplants. In this sense, nTreg cells could be used in immune therapies; for example, making use of their suppressor activity in the treatment of autoimmune or inflammatory diseases or in transplants, or suppressing said function in the treatment of cancer or AIDS. Hence, nTreg cells could be isolated from samples taken from patients having different diseases (rheumatoid arthritis, lupus, multiple sclerosis, AIDS, acute T cell lymphoma) to study their functionality, identify their number and proportion in relation to other lymphocytes, etc., or even in a more applied manner in personalised immune therapies: for example, purifying nTreg cells from the patient's blood, expanding that population of lymphocytes *in vitro,* and reinserting them again into the same individual for the purpose of controlling an autoimmune disease or preventing the rejection of a transplanted organ, for example.

For the identification and purification of nTreg cells it is essential to characterise their phenotype by means of molecular markers that can either be present or absent, or at least in a greater or lesser proportion, in these lymphocytes compared to effector T cells. Said molecular markers make it possible to define, within T CD4+ lymphocytes, which are nTreg cells and which are the "effector cells", and therefore to visualise them, count them or assess their presence in different tissues by means of cytometry or a microscope for example, or to purify them by means of FACS or magnetic systems for subsequent use, such as for example in clinical autologous transplants.

Human nTreg cells constitutively express on their surface proteins such as CD4, CD25/IL - 2Rα, CD27, LAG - 3, galectin - 1, CD38, neuropilin/RNP, OX - 40L, CD5, TNFR2, TGFβR1, GPR83, CD45RO, CTLA - 4, HLA - DR or GITR. This large number of markers generates a false sensation of certainty when it comes to fully defining and differentiating the population of nTreg cells from "effector" T lymphocytes. However, the markers described to date present several problems. In the first instance, due to the heterogeneity present in the population of nTreg cells, these markers are not present in 100 % of the nTreg cells. Secondly, these markers are not exclusive of this cell lineage. Hence, they may be absent in resting "effector" T cells, but be expressed in activated or memory "effector" cells; or on the contrary, they may be expressed by resting "effector" T cells, but lose said expression when they become activated or memory cells.

Most nTreg cells constitutively and strongly express CD25 and this expression is not lost with activation of the nTreg cells. However, "effector" T cells, initially CD4+CD25- when resting (a characteristic that as a matter of fact allows them to be distinguished from CD4+CD25+ nTreg cells) increase the expression of CD25 when activated (Baecher - Allan C. et al. J. Immunol. 2001, 167: 1245). The same problem occurs with GITR (McHugh R.S. et al. Immunity 2002, 16: 311), HLA - DR (Salgado F.J. et al. Immunol. Cell. Biol. 2002, 80: 138) or CTLA - 4/CD152 (Perkins D. et al. J. Immunol. 1996, 156: 4154), for example.

The nonexistent or low presence of CD127 on the cell surface has been associated to the existence of an nTreg phenotype in the lymphocytes of healthy donors (Liu W. et al. J. Exp. Med. 2006, 203: 1701; Seddiki N. J. Exp. Med. 2006, 203: 1693; Banham A.H. TRENDS Immunol 2006, 27: 541; WO 2007140472; WO 2007140472). However, the existence of inflammation (for example, in patients with rheumatoid arthritis) (Aerts N.E. et al. Cell. Immunol. 2008, 251: 109), makes T CD4+CD25+CD127- cells appear, which are not nTreg cells, but rather activated effector T cells.

FoxP3 represents a marker that is more associated with a regulatory cell phenotype, and its detection in permeabilised cells using anti-FoxP3 monoclonal antibodies is used by many researchers in order to identify these cells *in vivo.* However, the fact that FoxP3 is an intracellular marker means that it cannot be used in nTreg cell purification protocols without previously "killing off" the lymphocytes intended for "labelling" due to the need to permeabilise the cells. Furthermore, this marker is not entirely specific to nTreg cells, since fairly recent works indicate that, at least in humans, the activation of "effector" T cells CD4+CD25- induces a transient expression of FoxP3 (Tran D.Q. et al. Blood 2007, 110: 2983; Ziegler S.F. et al. Eur. J. Immunol 2007, 37: 21; Roncarolo M. - G and Gregori S. Eur. J. Immunol. 2008, 38: 901).

CD49d (α chain of VLA-4 integrin) is expressed in the majority of pro - inflammatory effector cells that produce IFNγ or IL-17, but not in nTreg cells (Kleinewietfeld M et al. Blood 2009, 113: 827; W02009047003). However, although it has been demonstrated that the phenotype CD4+CD25+CD49d-allows nTreg cells to be identified when the lymphocyte population is taken from a healthy individual, it has not been verified whether CD49d would allow nTreg cells to be characterised or purified in the case of lymphocytes taken from patients having inflammatory diseases or cancer.

Recently, the use of CD39 has been suggested as a marker of human nTregs. Using lymphocytes taken from patients with head and neck squamous cell carcinoma, its efficacy in identifying nTregs in the context of a cell activation or inflammatory condition has been proven. However, although CD4+CD39+ cells express low levels of CD127, these only contain 50-70% of FoxP3+ cells in healthy individuals, or 50-90% of FoxP3+ cells in patients with carcinoma. (Mandapathil et al. J. Immunol. Methods 2009, 346: 55). In this way, within CD4+CD39+ lymphocytes there are cells that express nonexistent or low levels of FoxP3 and that can be effector T lymphocytes.

Therefore, the lack of specificity affecting the markers described to date complicates the identification and purification of nTreg cells, especially in those samples taken from individuals having inflammatory diseases. In most protocols (Miltenyi Biotech, BD Biosciences, Invitrogen) purification of nTreg cells using magnetic systems is based on the CD4 and CD25 markers, and is carried out in two steps. The first one is a negative selection process wherein T cells that are not CD4+ are removed (erithrocytes, platelets, CD8+ T lymphocytes, T γδ lymphocytes, B lymphocytes, NK cells, macrophages, dendritic cells, granulocytes) using a mixture of biotinylated antibodies (for example anti-CD8, anti-CD11b/Mac1, anti-CD14, anti-CD16, anti-CD19, anti-CD36, anti-CD41a, anti-CD56, anti-CD123, anti-CD235a and γδTCR), which are recognised by microspheres coated with an anti-biotin antibody. In some protocols (Miltenyi) biotinylated anti-CD127 is added to the mixture of antibodies, to remove CD127+ cells (not nTreg cells). Cells that are not T CD4+, marked with the microspheres, are retained by the effect of a magnet; meanwhile, the CD4+ cells are not retained by the column and are collected in a tube. The second step is a positive selection process wherein the cells not retained in the previous step, with a CD4+ phenotype, are marked directly with microspheres coated with an anti-CD25 antibody, or indirectly with anti-APC microspheres that recognise any nTreg cell labelled with anti-CD25-APC antibodies. In either of the two cases, the cells are then passed through a separation system wherein the CD4+CD25+ nTregs are retained by the effect of the magnet, unlike CD4+CD25- cells. When the influence of the magnet is removed in a subsequent step, the nTreg cells are obtained pure.

Recently, a new protocol for the magnetic purification of nTregs has appeared on the market (Miltenyi Biotec) based on the use of the CD49d marker (Kleinewietfeld M et al. Blood 2009, 113: 827; W02009047003). This protocol also consists of two steps. The first one is a negative selection process wherein CD8+ and CD49d+ cells are magnetically marked with microspheres coated with anti-CD8 and anti-CD49d antibodies. The CD8+CD49d+ lymphocytes are retained in the column by the effect of the magnet, while the CD4+CD49d- cells pass through the column without being retained and are subsequently collected in a tube for use in the following step. In the second step, of positive selection, the CD49d- cells are marked with microspheres coated in anti-CD25. As they pass through the column the CD4+CD25+ nTreg cells are retained thanks to the effect of the magnet, whereas the CD4+CD25-pass freely through the column. Finally, the nTreg cells are eluted and collected in a tube for their subsequent use.

### EXPLANATION OF THE INVENTION

The present invention provides a method and a kit for the isolation or purification of nTreg cells from a biological sample, preferably from an individual having an inflammatory or autoimmune disease.

Both in research as well as in clinical practice there is enormous interest in purifying nTreg cells in a reliable manner. To this effect it is essential to have molecular markers that allow nTreg cells to be distinguished from effector T lymphocytes. However, the molecular markers described in the state of the art do not allow this distinction to be made when dealing with activated effector T lymphocytes, as occurs in the samples of individuals having inflammatory or autoimmune diseases. Therefore, there is a need to have molecular markers that can discriminate between nTreg cells and activated effector T lymphocytes. It is also desirable for such markers to be negative selection markers, in such a way that the nTreg cells can be purified free of marking, as required in cell therapy.

As shown in the examples of the present invention, the nonexistent or low levels of CD26 present on the cell surface of human nTreg cells compared to activated effector T lymphocytes, makes it possible to distinguish between these two populations. Unlike other negative selection markers described in the state of the art, the levels of CD26 increase in activated T lymphocytes. Thus nTreg cells can be purified using this marker to a high level of purity irrespective of the degree of activation of the starting lymphocyte population. The present invention thus resolves the problem of potential contamination with effector T lymphocytes present in current protocols, especially when analysing samples taken from patients with inflammatory diseases.

Therefore, one first aspect of the invention relates to a method for the isolation or purification of nTregs from an isolated biological sample (hereinafter, first method of the invention), comprising the following stages:
a) treating the aforesaid biological sample with an anti-CD26 antibody, and with at least one antibody selected from the list that comprises:
   i) an anti-CD25 antibody,
   ii) an anti-CD127 antibody,
   iii) an anti-CD49d antibody,
   or any combination thereof, and
b) separating the nTreg cells.

The expression "treating the biological sample", as used in the present description, implies that the cells comprising the biological sample come into physical contact with the antibodies, in such a way that the antibodies can interact with the cells that express the molecules specifically recognised by said antibodies.

The expression "separating", as used in the present description, refers to extracting using physical means a particular type of cells from a population consisting of at least two different cell types; for example, extracting nTreg cells from effector T cells, thereby allowing an enriched population of nTregs to be obtained. Separation can take place in one or more stages, which can be carried out consecutively.

The stages (a) and (b) of the first method of the invention can be carried out simultaneously. At the same time, stages (a) and (b) can be carried out a repeated number of times, either simultaneously or independently. In this way, stage (a) can be repeated an indeterminate number of times and/or stage (b) an indeterminate number of times, the number of repetitions of stage (a) not having to coincide with the number of repetitions of stage (b). For example, it is possible to carry out a first stage (a) and a first stage (b), and next, to carry out a second stage (a) and a second stage (b), and so on successively, with third, fourth, fifth, etc. stages (a) and (b). It is also possible, for example, to carry out several stages (a) followed by one or more stages (b).

In a preferred embodiment of the first method of the invention, steps (a) and (b) are carried out simultaneously. The advantage of carrying stages (a) and (b) simultaneously is that isolation of the nTreg cells is less labour intensive, simpler, faster and cheaper. The advantage of carrying out stages (a) and (b) a repeated number of times is that they can be used to separate different antibodies that allow both the positive selection as well as the negative selection of nTregs.

The expression "positive selection", as used in the present description, refers to the fact that wanted cells, for example, nTregs, are removed from the starting population by marking and capturing said cells, whereas the unwanted ones are left free of marking. Examples of antibodies that allow positive selection of nTreg cells are for example, but without limitation, the anti-CD4 antibody or anti-CD25 antibody.

The expression "negative selection", as used in the present description, refers to the fact that unwanted cells are removed from the cell repertory through their marking and capture, whereas the wanted ones, for example the nTreg cells, are left free of marking. Examples of antibodies that allow negative selection of nTreg cells are for example, but without limitation, the anti-CD26 antibody, the anti-CD127 antibody or the anti-CD49d antibody.

When in stage (a) an antibody is used that allows negative selection of the nTreg cells, in stage (b) the unwanted cells that express the molecule specifically recognised by said antibody are removed from the biological sample, leaving the wanted cells, for example the nTreg cells, free of marking. Therefore, in a preferred embodiment, in stage (b) of the first method of the invention the following cells are removed from the biological sample:
- CD26+ when in stage (a) the biological sample is treated with an anti-CD26 antibody; or
- CD127+ when in stage (a) the biological sample is treated with an anti-CD127 antibody; or
- CD49d+ when in stage (a) the biological sample is treated with an anti-CD49d antibody.

As demonstrated in the examples, the CD25+CD26- phenotype allows the population of nTreg cells to be differentiated from the population of CD25+/CD26+ effector T cells, irrespective of the degree of activation of the starting T cell population.

Therefore, in a preferred embodiment of the first method of the invention, in stage (a) the biological sample is treated with an anti-CD26 antibody and with an anti-CD25 antibody. In a more preferred embodiment, the first method of the invention comprises:
- a first stage (a), wherein the biological sample is treated with an anti-CD26 antibody,
- a first stage (b), wherein the CD26- cells are separated, through removal of the CD26+ cells from the biological sample;
- a second stage (a), wherein the biological sample is treated with an anti-CD25 antibody, and
- a second stage (b), wherein the CD25+CD26- nTreg cells are separated.

The interaction of an antibody with the molecule of the cell surface specifically recognised by this antibody can induce an unwanted intracellular signalling process, or activation of the complement system in the event of these cells being inserted in an individual. In other words, isolation of nTreg cells using only negative selection processes has the advantage of not triggering these processes.

For this reason, in another preferred embodiment of the first method of the invention, in stage (a) the biological sample is treated with an anti-CD26 antibody and with another or other additional antibodies that also allow negative selection of the nTreg cells. The biological sample can be treated, for example, sequentially with the anti-CD26 antibody and with another or other antibodies that allow negative selection, such as for example CD127 or CD49d, in such a way that stages (a) and (b) are carried out a repeated number of times. The biological sample can also be treated with these negative selection antibodies in a simultaneous manner, in such a way that the method is less labour - intensive, and easier, faster and cheaper.

In another preferred embodiment of the first method of the invention, in stage (a) the biological sample is treated with an anti-CD26 antibody and with an anti-CD127 antibody. Through treatment with the anti-CD26 antibody in stage (a) in stage (b) the CD26+ cells are removed from the biological sample and through treatment with the anti-CD127 antibody in stage (a) in stage (b) the CD127+ cells are removed from the biological sample; in this way CD127-CD26- nTreg cells are isolated. In a more preferred embodiment, in stage (a) the biological sample is treated simultaneously with an anti-CD26 antibody and with an anti-CD127 antibody.

In another preferred embodiment of the first method of the invention, in stage (a) the biological sample is treated with an anti-CD26 antibody and with an anti-CD49d antibody. Through treatment with the anti-CD26 antibody in stage (a) in stage (b) the CD26+ cells are removed from the biological sample and through treatment with the anti-CD49d antibody in stage (a) in stage (b) the CD49d+ cells are removed from the biological sample; in this way CD49d-CD26- nTreg cells are isolated. In a more preferred embodiment, in stage (a) the biological sample is treated simultaneously with an anti-CD26 antibody and with an anti-CD49d antibody.

In another preferred embodiment of the first method of the invention, in stage (a) the biological sample is treated with an anti-CD26 antibody, with an anti-CD127 antibody and with an anti-CD49d antibody. Through treatment with the anti-CD26 antibody in stage (a) in stage (b) the CD26+ cells are removed from the biological sample, through treatment with the anti-CD127 antibody in stage (a) in stage (b) the CD127+ cells are removed from the biological sample and through treatment with the anti-CD49d antibody in stage (a) in stage (b) the CD49d+ cells are removed from the biological sample; in this way CD127-CD49d-CD26- nTreg cells are isolated. In a more preferred embodiment, in stage (a) the biological sample is treated simultaneously with an anti-CD26 antibody, an anti-CD127 antibody and with an anti-CD49d antibody.

The advantage of combining antibodies that allow positive selection and antibodies that allow negative selection of the nTreg cells is that a more pure population of nTreg cells is isolated. Therefore, in a preferred embodiment, in stage (a) the biological sample is treated with an anti-CD26 antibody and with another or other additional antibodies that also allow negative selection of nTregs, such as for example CD127 or CD49d, and also with another or other additional antibodies that allow positive selection of the nTreg cells, such as for example, an anti-CD25 antibody.

In another preferred embodiment of the first method of the invention, in stage (a) the biological sample is treated with an anti-CD26 antibody, with an anti-CD25 antibody and with an anti-CD127 antibody. In a more preferred embodiment, the first method of the invention comprises:
- a first stage (a), wherein the biological sample is treated with an anti-CD26 antibody and an anti-CD127 antibody,
- a first stage (b), wherein the CD127-CD26- nTreg cells are separated, through removal of the CD127+ cells and CD26+ cells from the biological sample,
- a second stage (a), wherein the biological sample is treated with an anti-CD25 antibody, and
- a second stage (b), wherein the CD25+CD127-CD26- nTreg cells are separated.

In another preferred embodiment of the first method of the invention, in stage (a) the biological sample is treated with an anti-CD26 antibody, with an anti-CD25 antibody and with an anti-CD49d antibody. In a more preferred embodiment, the first method of the invention comprises:
- a first stage (a), wherein the biological sample is treated with an anti-CD26 antibody and an anti-CD49d antibody,
- a first stage (b), wherein the CD49d-CD26- nTreg cells are separated, through removal of the CD49d+ cells and CD26+ cells from the biological sample,

- a second stage (a), wherein the biological sample is treated with an anti-CD25 antibody, and
- a second stage (b), wherein the CD25+CD49d-CD26- nTreg cells are separated.

In another preferred embodiment of the first method of the invention, in stage (a) the biological sample is treated with an anti-CD26 antibody, with an anti-CD25 antibody, with an anti-CD127 antibody and with an anti-CD49d antibody. In a more preferred embodiment, the first method of the invention comprises:
- a first stage (a), wherein the biological sample is treated with an anti-CD26 antibody, an anti-CD127 antibody and an anti-CD49d antibody,
- a first stage (b), wherein the CD127-CD49d-CD26- nTreg cells are separated, through removal of the CD127+ cells, CD49d+ cells and CD26+ cells from the biological sample,
- a second stage (a), wherein the biological sample is treated with an anti-CD25 antibody, and
- a second stage (b), wherein the CD25+CD127-CD49d-CD26-nTreg cells are separated.

Also, stage (b) of the first method of the invention can comprise the removal from the biological sample of non CD4+ T cells. The advantage of removing these non CD4+ T cells from the sample is that the purity of the isolated nTreg cell population is higher. Therefore, in a preferred embodiment of the first method of the invention, in stage (b) non CD4+ T cells are removed from the biological sample.

Non CD4+ T cells can be removed from the sample through positive selection of the CD4+ cells. Therefore, in a preferred embodiment, in stage (a) of the first method of the invention the biological sample is treated with an anti-CD4 antibody; in such a way that in stage (b) non CD4+ T cells are removed and nTreg cells that are CD4+ are captured (separated).

At the same time, non CD4+ T cells can also be removed from the biological sample through negative selection, using for this purpose one or more antibodies that specifically recognise T cells that are not CD4+. Therefore, in a preferred embodiment, in stage (a) of the first method of the invention the biological sample is treated with an antibody that recognises a molecule present in non CD4+ T cells. In a more preferred embodiment in stage (a) of the first method of the invention the biological sample is treated with an antibody that recognises a molecule present in non CD4+ T cells selected from the list that comprises the following antibodies: anti-CD8, anti-CD11b/Mac1, anti-CD14, anti-CD16, anti-CD19, anti-CD36, anti-CD41a, anti-CD56, anti-CD123, anti-CD235a and anti-γδTCR, or with any combination thereof.

For example, in another preferred embodiment of the first method of the invention, in stage (a) the biological sample is treated with an anti-CD8 antibody, with an anti-CD26 antibody, with an anti-CD127 antibody and with an anti-CD49d antibody. Through treatment with the anti-CD8 antibody in stage (a) in stage (b) some T and NK cells that are CD8+ are removed from the biological sample, through treatment with the anti-CD26 antibody in stage (a) in stage (b) CD26+ cells are removed from the biological sample, through treatment with the anti-CD127 antibody in stage (a) in stage (b) CD127+ cells are removed from the biological sample and through treatment with the anti-CD49d antibody in stage (a) in stage (b) CD49d+ cells are removed from the biological sample (T and B lymphocytes, as well as weakly in macrophages); in this way CD4+ CD127-CD49d-CD26- nTreg cells are isolated. In a more preferred embodiment, the biological sample is treated simultaneously with anti-CD8, anti-CD26, anti-CD127 and anti-CD49d.

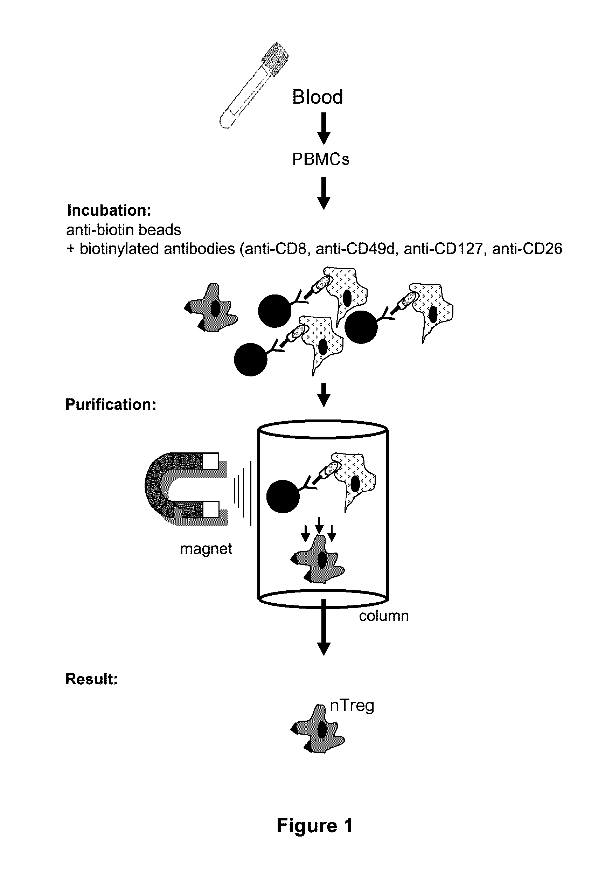

**Figure 1** is a diagram showing a preferred embodiment of the first method of the invention. The protocol starts out with venous blood from a normal individual or from a patient having an inflammatory disease. From this blood, human peripheral blood mononuclear cells (PBMCs) are obtained. The PBMCs are incubated using magnetic beads coated with an anti - biotin antibody, which recognise the biotinylated antibodies anti-CD8, anti-CD26, anti-CD127 and anti-CD49d with which the cells are also incubated. The PBMCs are made to pass through a column or support, which retains due to the magnet effect only the non regulatory cells "labelled" with the magnetic beads. However, the nTregs are not captured and are obtained free of bound antibodies.

In a more preferred embodiment of the first method of the invention, in stage (a) an anti-CD45RO antibody is also used, in such a way that the isolated nTregs are CD45RA+. The advantage of using said anti-CD45RO antibody as an additional antibody in the first method of the invention is that a specific population of nTreg cells is isolated, known as "naïve" CD45RA+ T nTregs cells, which are very effective and pure.

In another more preferred embodiment of the first method of the invention, in stage (a) an anti-CD45RA antibody is also used, in such a way that the isolated nTregs are CD45RO+. The advantage of using said anti-CD45RA antibody as an additional antibody in the first method of the invention is that a specific population of nTreg cells is isolated, known as memory CD45RO+ T nTregs cells, which are very effective and pure.

The term "antibody", as used in the present description, refers to immunoglobulin molecules and immunologically active portions of immunoglobulin molecules, in other words, molecules containing one antigen binding site that binds specifically (immunoreacts) with a protein. Examples of portions of immunologically active immunoglobulin molecules, include fragments F(ab) and F(ab')2 which may be generated by treating the antibody with an enzyme such as pepsin. The antibodies may be polyclonal (these typically include different antibodies directed against different determinants or epitopes) or monoclonal (directed against a single determinant in the antigen). The monoclonal antibody may be biochemically altered by genetic manipulation or may be synthetic, possibly lacking the antibody in its totality or in parts, of portions that are not necessary for the recognition of the protein and being substituted by others which communicate additional advantageous properties to the antibody. The antibody may also be recombinant, chimeric, humanised, synthetic or any combination of the above. A "recombinant polypeptide or antibody" (rAC) is an antibody that has been produced in a host cell that has been transformed or transfected with the nucleic acid encoding the polypeptide, or produces the polypeptide as a result of homologous recombination. The expression "anti-X antibody" refers to an antibody capable of specifically recognising a particular protein X; for example, an anti-CD26 antibody is an antibody capable of specifically recognising protein CD26. Antibodies that can be used in the present invention are known in the state of the art, such as the ones described, but without limitation, in the examples of the present description.

The antibodies used in the first method of the invention may be marked or immobilised. The advantage of using marked or immobilised antibodies in respect of using unmarked or non - immobilised ones is that it helps to adapt them to standard isolation techniques and they can be used with standard equipment. Therefore, in a preferred embodiment of the first method of the invention, at least one of the antibodies used in stage (a) is marked or immobilised.

The term "marked", as used in the present description, refers to the fact that the antibody is conjugated to a label. The state of the art knows a high number of labels that can be conjugated to an antibody. Examples of labels that can be used to mark an antibody are, but without limitation, radioisotopes [for example, ³²P, ³⁵S o ³H], fluorescent or luminescent markers [for example, fluorescein (FITC), rhodamine, *texas red,* phycoerythrin (PE), allophycocyanin, 6-carboxyfluorescein (6-FAM), 2',7'-dimethoxy-4',5'-dichloro-6-carboxyfluorescein (JOE), 6-carboxy-X-rhodamine (ROX), 6-carboxy-2',4',7',4,7 -hexachlorofluorescein (HEX), 5-carboxyfluorescein (5-FAM) or N,N,N',N'-tetramethyl-6-carboxyrhodamine (TAMRA)]; fragments of antibodies [for example, fragments F(ab)2)], affinity labels [for example, biotin, avidin, agarose, bone morphogenetic protein (BMP), haptens], enzymes or enzyme substrates [for example, alkaline phosphatase (AP) and horse radish peroxidase (HRP)].

In a preferred embodiment of the first method of the invention, at least one of the antibodies used in stage (a) is marked. In a more preferred embodiment of the first method of the invention, at least one of the antibodies used in stage (a) is marked and also uniformly; the advantage of uniformly marking the antibodies is that all the antibodies can be detected at the same time. In a preferred embodiment of the first method of the invention, at least one of the antibodies used in stage (a) is marked with a label selected from the list that comprises: a radioisotope, a fluorescent or luminescent marker, an antibody, a fragment of antibody, an affinity label, an enzyme and an enzyme substrate.

The term "immobilised", as used in the present description, refers to the fact that the antibody may be bound to a support without losing its activity. Preferably, the support may be a matrix surface, (for example, a nylon matrix), a microtest plate (with 96 wells for example) or similar plastic support, or beads (*spheres,* for example *spheres* of agarose or small superparamagnetic microspheres composed of biodegradable matrices).

In a preferred embodiment of the first method of the invention, at least one of the antibodies used in stage (a) is immobilised. In a more preferred embodiment of the first method of the invention, at least one of the antibodies used in stage (a) is immobilised in a support selected from the list that comprises: a nylon matrix, a plastic support or in beads.

In the state of the art several methods are known wherein the separation of nTreg cells can be achieved in stage (b) of the first method of the invention. In a preferred embodiment of the first method of the invention, stage (b) is carried out by means of at least one method selected from the list that comprises: centrifugation, FACS/fluorescence activated cell separation, magnetic cell separation, column-based immunological separation, cell adhesion, complement-mediated lysis, or any combination thereof. In a more preferred embodiment of the first method of the invention the separation is achieved using methods such as FACS or magnetic systems.

The expression "column-based immunological separation", as used in the present description, refers to a method for classifying cells wherein the antibodies used in the first method of the invention are bound to chromatography column resins and in this way are used to bind the cells that express on their surface the molecule recognised by the specific antibody.

The expression "fluorescence-activated cell separation" or FACS refers, as used in the present description, to a method that allows cells to be classified following their prior joining to antibodies that are bound to fluorochromes and which are specific to certain molecules found on the cell surface.

The expression "magnetic systems", as used in the present description, refers to cell purification methods wherein antibodies are used that specifically recognise molecules expressed on the surface of certain cells and that are bound to magnetic spheres or are recognised by other molecules (antibodies, etc.) bound to said magnetic spheres, in such a way that the cells can be retained by the effect of a magnetic field created by a magnet.

The term "isolated biological sample", as used in the present description refers, but is not limited to, cell populations and to the biological tissues and/or fluids of an individual, obtained using any means known to a person skilled in the art to serve this purpose. Preferably, the isolated biological sample comprises cells and, more preferably, lymphocytes. Examples of tissues that comprise lymphocytes are, but without limitation, the spleen, thymus, lymph node, bone marrow, Peyer's patch and tonsil. Examples of fluids that comprise lymphocytes are, but without limitation, synovial fluid, lymph, cerebrospinal fluid, urine, pleural fluid, pericardial fluid, peritoneal fluid, amniotic fluid or discharge (for example, nasopharyngeal, vaginal, urethral, conjunctival or eye) or blood. Examples of cell populations that comprise lymphocytes are, but without limitation, leukocyte concentrate (*buffy coat*), peripheral blood mononuclear cells (PBMCs), peripheral blood lymphocytes (PBLs) or cell lines. Therefore, in a preferred embodiment, the isolated biological sample is selected from the list that comprises: spleen, thymus, lymph node, bone marrow, Peyer's patch, tonsil, synovial fluid, lymph, cerebrospinal fluid, urine, pleural fluid, pericardial fluid, peritoneal fluid, amniotic fluid, discharge, blood, leukocyte concentrate, peripheral blood mononuclear cells, peripheral blood lymphocytes or cell lines. In a more preferred embodiment, the isolated biological sample is selected from the list that comprises: blood, leukocyte concentrate, peripheral blood mononuclear cells, peripheral blood lymphocytes, spleen or lymph node.

The term "individual", as used in the description, refers to an animal, preferably a mammal, and more preferably, a human being. Therefore, the first method of the invention is a method for the isolation of the nTreg cells of an animal, more preferably the nTreg cells of a mammal, and even more preferably, human nTreg cells.

The main advantage of using CD26 as a marker for the isolation of nTreg cells is that it allows purification of this cell type irrespective of the degree of activation of the lymphocytes of the starting biological sample of the first method of the invention, and is therefore very suitable for isolating biological samples from an individual that may have activated lymphocytes. Therefore, in a preferred embodiment, the biological sample is taken from an individual having an autoimmune disease, an allergic disease, an inflammatory disease, an infectious disease or a parasitic disease.

The term "inflammatory disease", as used in the present description refers to a disease in which injuries are produced by an immune reaction or an inflammatory reaction of the body. Some examples of inflammatory diseases are, for example, but without limitation, conjunctivitis, iritis, uveitis, retinitis, otitis mastoiditis, rhinitis, labyrinthitis, sinusitis, pharyngitis, tonsillitis, bronchitis, pneumonia, bronchopneumonia, pleuritis, mediastinitis, endocarditis, thrombophlebitis, polyarteritis, nephritis, cystitis, stomatitis, esophagitis, gastritis, colitis, appendicitis, hepatitis, cholecystitis, pancreatitis, peritonitis, thyroiditis, dermatitis, encephalitis, meningitis, neuromyelitis optica, polyneuritis, polymyositis, fibrodysplasia ossificans progressiva, osteoarthritis or rheumatoid arthritis. Some inflammatory diseases, such as rheumatoid arthritis are disorders of an autoimmune nature.

The term "autoimmune disease", as used in the present description, refers to a pathological process that produces an immune response against one's own body. Examples of autoimmune diseases include, but without limitation, Crohn's disease, pernicious anaemia, type I diabetes, Addison's disease, Coeliac disease, Graves' disease, multiple sclerosis, systemic lupus erythematosus, myasthenia gravis, Reiter syndrome, Sjogren's syndrome, Hashimoto's thyroiditis, scleroderma, Goodpasture's syndrome, Wegener's granulomatosis, polymyalgia rheumatica or rheumatoid arthritis.

The terms "allergic disease" or "allergy", as used in the present description, refer to a hypersensitive reaction to a particular substance (or allergen) mediated by immune mechanisms. Depending on the type of allergen it may be a question of, for example, but without limitation, allergy to flower pollens or to other vegetable products, a drug allergy, food allergy, allergy to the epidermal substances of animals, allergy to bacterial products or infective allergy, allergy to dust mites, allergy to metals, allergy to latex or allergy to insect bites.

The term "infectious disease", as used in the present description, refers to the clinical manifestation resulting from an infection caused by a microorganism, such as for example, but without limitation, a bacterium, a fungus, a virus or a protozoon.

The term "parasitic disease", as used in the present description refers to a clinical manifestation resulting from an infection or infestation caused by a protozoon, a helminth or an arthropod.

A second aspect of the present invention relates to a kit (hereinafter, first kit of the invention), that comprises:
a) an anti-CD26 antibody, and
b) at least one antibody selected from the list comprising:
   i) an anti-CD25 antibody,
   ii) an anti-CD127 antibody, and
   iii) an anti-CD49d antibody.

In a preferred embodiment, the first kit of the invention comprises an anti-CD26 antibody and an anti-CD25 antibody. In another preferred embodiment, the first kit of the invention comprises an anti-CD26 antibody and an anti-CD127 antibody. In another preferred embodiment of the first method of the invention, the first kit of the invention comprises an anti-CD26 antibody and an anti-CD49d antibody. In another preferred embodiment, the first kit of the invention comprises an anti-CD26 antibody, an anti-CD127 antibody and an anti-CD49d antibody. In another preferred embodiment, the first kit of the invention comprises an anti-CD26 antibody, an anti-CD25 antibody and an anti-CD127 antibody. In another preferred embodiment, the first kit of the invention comprises an anti-CD26 antibody, an anti-CD25 antibody and an anti-CD49d antibody. In another preferred embodiment, the first kit of the invention comprises an anti-CD26 antibody, an anti-CD25 antibody, an anti-CD127 antibody and an anti-CD49d antibody.

In a more preferred embodiment, the first kit of the invention also comprises at least one antibody that recognises a molecule present in cells that are not CD4+ T lymphocytes. In an even more preferred embodiment, the first kit of the invention also comprises at least one antibody selected from the list comprising: anti-CD8, anti-CD11b/Mac1, anti-CD14, anti-CD16, anti-CD19, anti-CD36, anti-CD41 a, anti-CD56, anti-CD123, anti-CD235a and anti- γδTCR.

In a preferred embodiment, at least one of the antibodies of the first kit of the invention is marked or immobilised. In a preferred embodiment, at least one of the antibodies of the first kit of the invention is marked with a label selected from the list comprising: a radioisotope, a fluorescent or luminescent marker, an antibody, an antibody fragment, an affinity label, an enzyme and an enzyme substrate. In a preferred embodiment, at least one of the antibodies of the first kit of the invention is immobilised in a nylon matrix, in a plastic support or in beads.

In a preferred embodiment, the first kit of the invention also comprises at least one element necessary for separating the nTreg cells. Hence, the first kit of the invention may comprise elements that allow nTregs to be separated by means of, for example, but without limitation, a method selected from the list comprising: centrifugation, fluorescence activated cell separation/FACS, magnetic cell separation, column based immunological separation, cellular adhesion or complement - mediated lysis. In a preferred embodiment, the first kit of the invention also comprises at least one element necessary for separating the nTreg cells selected from the list comprising: columns, column supports, fraction collection tubes, magnets, meshes for removing cell aggregates, solutions containing complement factors, isotonic wash buffers, cell enrichment/separation solutions (for example, Ficoll-Paque™), superparamagnetic beads, plastic plates or supports with antibodies or other bound molecules (for example, streptavidin), components that allow dead cells to be removed before the purification process or solutions of propidium iodide or 7-AAD for the exclusion of dead cells during evaluation by flow cytometry of the purity of the nTregs once these have been isolated. Also, antibodies bound to fluorochromes (for example, CD4 - FITC, FoxP3 - PE and CD25 - APC) and cell permeabilisation solutions to evaluate by flow cytometry the purity of the nTregs once these have been purified.

The kit may also contain any other reagent necessary for carrying out the first method of the invention such as, for example, but without limitation, positive and/or negative controls, isotonic washes, anti - contamination agents, protein degradation inhibitors, etc. Also, the kit may include and support and/or container necessary for execution and optimisation. Preferably, the kit also comprises the instructions for carrying out the first method of the invention.

A third aspect of the present invention relates to the use of the first kit of the invention to isolate or purify nTreg cells from an isolated biological sample. Preferably, the biological sample is taken from an individual having an autoimmune disease, an allergic disease, an inflammatory disease, an infectious disease or a parasitic disease.

A fourth aspect of the present invention relates to a method for the identification of nTreg cells in an isolated biological sample (hereinafter, second method of the invention), which comprises the following stages:
a) detecting in the biological sample the expression product of the *CD26* gene, and the expression product of at least one of the genes selected of the list that comprises: *CD25, FoxP3, CD127, CD49d,* or any combination thereof,
b) comparing the amount of the expression product of the genes detected in stage (a) with a reference amount, and
c) assigning the identification of the nTreg cells to the comparison made in stage (b), when the amount detected of the expression product of the *CD26* gene is significantly lower than the reference amount for the *CD26* gene, and when:
   - the amount detected of the expression product of the *CD25* gene is significantly higher than the reference amount for the *CD25* gene, or
   - the amount detected of the expression product of the *FoxP3* gene is significantly higher than the reference amount for the *FoxP3* gene, or
   - the amount detected of the expression product of the *CD127* gene is significantly lower than the reference amount for the *CD127* gene, or
   - the amount detected of the expression product of the *CD49d* gene is significantly lower than the reference amount for the *CD49d* gene, or
      any combination thereof.

The second method of the invention allows identification of nTreg cells in an isolated biological sample. In addition to the steps specified above it may comprise other additional steps, for example related to pre - treatment of the sample or evaluation of the results obtained by means of this method. Preferably, steps (a) and/or (b) of the second method of the invention may be totally or partially automated, for example by means of a robotic sensor device to detect the amount in step (a) or computerised comparison in step (b).

As demonstrated in the examples of the present description, nTreg cells can be identified within a biological sample through detection of the amount of the expression product of the *CD26* gene and one of the genes *CD25, FoxP3, CD127* or *CD49d.* For the identification of nTreg cells it is also possible to use, in addition to detection of the expression product of the CD26 gene, detection of the amount of product of any of the combinations of the genes *CD25, FoxP3, CD127* or *CD49d,* for example, of two of these genes, of three of these genes, or of all four genes. The advantage of detecting the expression product of a lower number of genes is that identification of the nTreg cells is less labour - intensive, easier, quicker and cheaper. The advantage of detecting the expression product of a higher number of genes is that the identification of the nTreg cells is more reliable.

As demonstrated in the examples, the CD25 + CD26 - phenotype allows the nTreg cell population to be differentiated from the effector T cell population, irrespective of the degree of activation of the starting population of T cells. Therefore, in a preferred embodiment, in stage (a) of the second method of the invention the expression product of genes *CD26* and *CD25* is detected in such a way that in stage (c) identification of the nTreg cells is assigned when:
- the amount detected of the expression product of the *CD26* gene is significantly lower than the reference amount for the *CD26* gene, and
- the amount detected of the expression product of the *CD25* gene is significantly higher than the reference amount for the *CD25* gene.

In other words, when in stage (a) of the second method of the invention the expression product of the genes *CD26* and *CD25* is detected, in stage (c) the CD25+CD26- phenotype is indicative of the presence of nTreg cells in the biological sample.

Likewise, as demonstrated in the examples, the FoxP3+CD26-phenotype allows the nTreg cell population to be differentiated from the effector T cell population, irrespective of the degree of activation of the starting population of T cells. Therefore, in another preferred embodiment, in stage (a) of the second method of the invention the expression product of the genes *CD26* and *FoxP3* is detected in such a way that in stage (c) identification of the nTreg cells is assigned when:
- the amount detected of the expression product of the *CD26* gene is significantly lower than the reference amount for the *CD26* gene, and
- the amount detected of the expression product of the *FoxP3* gene is significantly higher than the reference amount for the *FoxP3* gene.

In other words, when in stage (a) of the second method of the invention the expression product of genes *CD26* and *FoxP3* is detected, in stage (c) the FoxP3+CD26- phenotype is indicative of the presence of nTreg cells in the biological sample.

For the identification of nTreg cells, the negative selection marker CD26 can be used and at least one positive selection marker such as, for example, CD25 or FoxP3. However, it is possible to use in addition to the negative selection marker CD26, at least one other negative selection marker such as, for example CD127 or CD49d.

The expression "positive selection marker", as used in the present description refers to the expression product of a gene that is detected in the wanted cells, for example, nTreg cells, whereas it is not detected in unwanted cells. Examples of positive selection markers of nTreg cells are, for example, but without limitation, the expression products of the genes *CD4, CD25* or *FoxP3.*

The expression "negative selection marker" as used in the present description, refers to the expression product of a gene that is detected in unwanted cells, whereas it is not detected in the wanted cells, for example, nTreg cells. Examples of negative selection markers of nTreg cells are, for example, but without limitation, the expression products of the genes *CD26, CD127* or *CD49d.*

In another preferred embodiment, in stage (a) of the second method of the invention the expression product of the genes *CD26* and *CD49d* is detected, in such a way that in stage (c) identification of the nTreg cells is assigned when:
- the amount detected of the expression product of the *CD26* gene is significantly lower than the reference amount for the *CD26* gene, and
- the amount detected of the expression product of the *CD49d* gene is significantly lower than the reference amount for the *CD49d* gene.

In other words, when in stage (a) of the second method of the invention the expression product of genes *CD26* and *CD49d* is detected, in stage (c) the CD49d-CD26- phenotype is indicative of the presence of nTreg cells in the biological sample.

In another preferred embodiment, in stage (a) of the second method of the invention the expression product of genes *CD26* and *CD127* is detected, in such a way that in stage (c) identification of the nTreg cells is assigned when:
- the amount detected of the expression product of the *CD26* gene is significantly lower than the reference amount for the *CD26* gene, and
- the amount detected of the expression product of the *CD127* gene is significantly lower than the reference amount for the *CD127* gene.

In other words, when stage (a) of the second method of the invention detects the expression product of genes *CD26* and *CD127* in stage (c) the CD127-CD26- phenotype is indicative of the presence of nTreg cells in the biological sample.

nTreg cells can be identified within a biological sample through detection of the amount of the expression product of the *CD26* gene and of two or three of the genes *CD25, FoxP3, CD127* or *CD49d.* Similarly, nTreg cells can be identified by detecting the amount of the expression product of the genes *CD26, CD25, FoxP3, CD127* or *CD49d.* The advantage of detecting the expression product of a greater number of genes is that identification of the nTreg cells is more reliable.

In another preferred embodiment, in stage (a) of the second method of the invention the expression product of genes *CD26, CD25* and *FoxP3* is detected in such a way that in stage (c) identification of the nTreg cells is assigned when:
- the amount detected of the expression product of the *CD26* gene is significantly lower than the reference amount for the *CD26* gene,
- the amount detected of the expression product of the *CD25* gene is significantly higher than the reference amount for the *CD25* gene, and
- the amount detected of the expression product of the *FoxP3* gene is significantly higher than the reference amount for the *FoxP3* gene.

In other words, when stage (a) of the second method of the invention detects the expression product of the genes *CD26, CD25* and *FoxP3* in stage (c) the CD25+FoxP3+CD26- phenotype is indicative of the presence of nTreg cells in the biological sample.

In another preferred embodiment, in stage (a) of the second method of the invention the expression product of genes *CD26, CD25* and *CD127* is detected in such a way that in stage (c) identification of the nTreg cells is assigned when:
- the amount detected of the expression product of the *CD26* gene is significantly lower than the reference amount for the *CD26* gene,
- the amount detected of the expression product of the *CD25* gene is significantly higher than the reference amount for the *CD25* gene, and
- the amount detected of the expression product of the *CD127* gene is significantly lower than the reference amount for the *CD127* gene.

In other words, when stage (a) of the second method of the invention detects the expression product of genes *CD26, CD25* and *CD127* in stage (c) the CD25+CD127-CD26- phenotype is indicative of the presence of nTreg cells in the biological sample.

In another preferred embodiment, in stage (a) of the second method of the invention the expression product of genes *CD26, FoxP3* and *CD127* is detected in such a way that in stage (c) identification of the nTreg cells is assigned when:
- the amount detected of the expression product of the *CD26* gene is significantly lower than the reference amount for the *CD26* gene,
- the amount detected of the expression product of the *FoxP3* gene is significantly higher than the reference amount for the *FoxP3* gene, and
- the amount detected of the expression product of the *CD127* gene is significantly lower than the reference amount for the *CD127* gene.

In other words, when stage (a) of the second method of the invention detects the expression product of genes *CD26, FoxP3* and *CD127* in stage (c) the FoxP3+CD127-CD26- phenotype is indicative of the presence of nTreg cells in the biological sample.

In another preferred embodiment, in stage (a) of the second method of the invention the expression product of genes *CD26, CD25* and *CD49d* is detected, in such a way that in stage (c) identification of the nTreg cells is assigned when:
- the amount detected of the expression product of the *CD26* gene is significantly lower than the reference amount for the *CD26* gene,
- the amount detected of the expression product of the *CD25* gene is significantly higher than the reference amount for the *CD25* gene, and
- the amount detected of the expression product of the *CD49d* gene is significantly lower than the reference amount for the *CD49d* gene.

In other words, when stage (a) of the second method of the invention detects the expression product of genes *CD26, CD25* and *CD49d* in stage (c) the CD25+CD49d-CD26- phenotype is indicative of the presence of nTreg cells in the biological sample.

In another preferred embodiment, in stage (a) of the second method of the invention the expression product of genes *CD26, FoxP3* and *CD49d* is detected, in such a way that in stage (c) identification of the nTreg cells is assigned when:
- the amount detected of the expression product of the *CD26* gene is significantly lower than the reference amount for the *CD26* gene,
- the amount detected of the expression product of the *FoxP3* gene is significantly higher than the reference amount for the *FoxP3* gene, and
- the amount detected of the expression product of the *CD49d* gene is significantly lower than the reference amount for the *CD49d* gene.

In other words, when stage (a) of the second method of the invention detects the expression product of genes *CD26, FoxP3* and *CD49d* in stage (c) the FoxP3+CD49d-CD26- phenotype is indicative of the presence of nTreg cells in the biological sample.

In another preferred embodiment, in stage (a) of the second method of the invention the expression product of genes *CD26, CD127* and *CD49d* is detected, in such a way that in stage (c) identification of the nTreg cells is assigned when:
- the amount detected of the expression product of the *CD26* gene is significantly lower than the reference amount for the *CD26* gene,
- the amount detected of the expression product of the *CD127* gene is significantly lower than the reference amount for the *CD127* gene and
- the amount detected of the expression product of the *CD49d* gene is significantly lower than the reference amount for the *CD49d* gene.

In other words, when stage (a) of the second method of the invention detects the expression product of genes *CD26, CD127* and *CD49d* in stage (c) the CD127-CD49d-CD26- phenotype is indicative of the presence of nTreg cells in the biological sample.

In another preferred embodiment, in stage (a) of the second method of the invention the expression product of genes *CD26, CD25, FoxP3* and *CD127* is detected, in such a way that in stage (c) identification of the nTreg cells is assigned when:
- the amount detected of the expression product of the *CD26* gene is significantly lower than the reference amount for the *CD26* gene,
- the amount detected of the expression product of the *CD25* gene is significantly higher than the reference amount for the *CD25* gene,
- the amount detected of the expression product of the *FoxP3* gene is significantly higher than the reference amount for the *FoxP3* gene, and
- the amount detected of the expression product of the *CD127* gene is significantly lower than the reference amount for the *CD127* gene.

In other words, when stage (a) of the second method of the invention detects the expression product of genes *CD25, CD26, FoxP3* and *CD127* in stage (c) the CD25+FoxP3+CD127-CD26- phenotype is indicative of the presence of nTreg cells in the biological sample.

In another preferred embodiment, in stage (a) of the second method of the invention the expression product of genes *CD26, CD25, FoxP3* and *CD49d* is detected, in such a way that in stage (c) identification of the nTreg cells is assigned when:
- the amount detected of the expression product of the *CD26* gene is significantly lower than the reference amount for the *CD26* gene,
- the amount detected of the expression product of the *CD25* gene is significantly higher than the reference amount for the *CD25* gene,
- the amount detected of the expression product of the *FoxP3* gene is significantly higher than the reference amount for the *FoxP3* gene, and
- the amount detected of the expression product of the *CD49d* gene is significantly lower than the reference amount for the *CD49d* gene.

In other words, when stage (a) of the second method of the invention detects the expression product of genes *CD26, CD25, FoxP3* and *CD49d* in stage (c) the CD25+FoxP3+CD49d-CD26- phenotype is indicative of the presence of nTreg cells in the biological sample.

In another preferred embodiment, in stage (a) of the second method of the invention the expression product of genes *CD26, CD25,* CD*127* and *CD49d* is detected in such a way that in stage (c) identification of the nTreg cells is assigned when:
- the amount detected of the expression product of the *CD26* gene is significantly lower than the reference amount for the *CD26* gene,
- the amount detected of the expression product of the *CD25* gene is significantly higher than the reference amount for the *CD25* gene,
- the amount detected of the expression product of the *CD127* gene is significantly lower than the reference amount for the *CD127* gene, and
- the amount detected of the expression product of the *CD49d* gene is significantly lower than the reference amount for the *CD49d* gene.

In other words, when stage (a) of the second method of the invention detects the expression product of genes *CD26, CD25, CD127* and *CD49d* in stage (c) the CD25+CD127-CD49d-CD26- phenotype is indicative of the presence of nTreg cells in the biological sample.

In another preferred embodiment, in stage (a) of the second method of the invention the expression product of genes *CD26, FoxP3,* CD*127* and *CD49d* is detected, in such a way that in stage (c) identification of the nTreg cells is assigned when:
- the amount detected of the expression product of the *CD26* gene is significantly lower than the reference amount for the *CD26* gene,
- the amount detected of the expression product of the *FoxP3* gene is significantly higher than the reference amount for the *FoxP3* gene,
- the amount detected of the expression product of the *CD127* gene is significantly lower than the reference amount for the *CD127* gene, and
- the amount detected of the expression product of the *CD49d* gene is significantly lower than the reference amount for the *CD49d* gene.

In other words, when stage (a) of the second method of the invention detects the expression product of genes *CD26, FoxP3, CD127* and *CD49d* in stage (c) the FoxP3+CD127-CD49d-CD26- phenotype is indicative of the presence of nTreg cells in the biological sample.

In another preferred embodiment, in stage (a) of the second method of the invention the expression product of genes *CD26, CD25, FoxP3, CD127* and *CD49d* is detected, in such a way that in stage (c) identification of the nTreg cells is assigned when:
- the amount detected of the expression product of the *CD26* gene is significantly lower than the reference amount for the *CD26* gene,
- the amount detected of the expression product of the *CD25* gene is significantly higher than the reference amount for the *CD25* gene,
- the amount detected of the expression product of the *FoxP3* gene is significantly higher than the reference amount for the *FoxP3* gene,
- the amount detected of the expression product of the *CD127* gene is significantly lower than the reference amount for the *CD127* gene, and
- the amount detected of the expression product of the *CD49d* gene is significantly lower than the reference amount for the *CD49d* gene.

In other words, when stage (a) of the second method of the invention detects the expression product of genes *CD26, CD25, FoxP3, CD127* and *CD49d* in stage (c) the CD25+FoxP3+CD127-CD49d-CD26- phenotype is indicative of the presence of nTreg cells in the biological sample.

In a preferred embodiment, in stage (a) of the second method of the invention the amount of the expression product of the *CD4* gene is also detected, in such a way that in stage (c) identification of the nTreg cells is assigned when the amount detected of the expression product of the *CD4* gene is significantly higher than the reference amount for the *CD4* gene, when the amount detected of the expression product of the *CD26* gene is significantly lower than the reference amount for the *CD26* gene, and when:
- the amount detected of the expression product of the *CD25* gene is significantly higher than the reference amount for the *CD25* gene, or
- the amount detected of the expression product of the *FoxP3* gene is significantly higher than the reference amount for the *FoxP3* gene, or
- the amount detected of the expression product of the *CD127* gene is significantly lower than the reference amount for the *CD127* gene, or
- the amount detected of the expression product of the *CD49d* gene is significantly lower than the reference amount for the *CD49d* gene, or
- any combination thereof.

In other words, the CD4+ criterion is incorporated to any of the phenotypes described above as indicative of nTreg cells; what follows is a description of different alternative embodiments of the second method of the invention that take this criterion into account. When stage (a) of the second method of the invention detects the expression product of genes *CD4, CD26* and *CD25,* in stage (c) the CD4+CD25+CD26- phenotype is indicative of the presence of nTreg cells in the biological sample. When stage (a) of the second method of the invention detects the expression product of genes *CD4, CD26* and *FoxP3,* in stage (c) the CD4+FoxP3+CD26- phenotype is indicative of the presence of nTreg cells in the biological sample. When stage (a) of the second method of the invention detects the expression product of genes *CD4, CD26* and *CD49d* in stage (c) the CD4+CD49d-CD26- phenotype is indicative of the presence of nTreg cells in the biological sample. When stage (a) of the second method of the invention detects the expression product of genes *CD4, CD26* and *CD127* in stage (c) the CD4+CD127-CD26- phenotype is indicative of the presence of nTreg cells in the biological sample. When stage (a) of the second method of the invention detects the expression product of genes *CD4, CD26, CD25* and *FoxP3* in stage (c) the CD4+CD25+FoxP3+CD26- phenotype is indicative of the presence of nTreg cells in the biological sample. When stage (a) of the second method of the invention detects the expression product of genes *CD4, CD26, CD25* and *CD127* in stage (c) the CD4+CD25+CD127-CD26- phenotype is indicative of the presence of nTreg cells in the biological sample. When stage (a) of the second method of the invention detects the expression product of genes *CD4, CD26, FoxP3* and *CD127* in stage (c) the CD4+FoxP3+CD127-CD26- phenotype is indicative of the presence of nTreg cells in the biological sample. When stage (a) of the second method of the invention detects the expression product of genes *CD4, CD26, CD25* and *CD49d* in stage (c) the CD4+CD25+CD49d-CD26- phenotype is indicative of the presence of nTreg cells in the biological sample. When stage (a) of the second method of the invention detects the expression product of genes *CD4, CD26, FoxP3* and *CD49d* in stage (c) the CD4+FoxP3+CD49d-CD26-phenotype is indicative of the presence of nTreg cells in the biological sample. When stage (a) of the second method of the invention detects the expression product of genes *CD4, CD26, CD127* and *CD49d* in stage (c) the CD4+CD127-CD49d-CD26- phenotype is indicative of the presence of nTreg cells in the biological sample. When stage (a) of the second method of the invention detects the expression product of genes CD4, *CD25, CD26, FoxP3* and *CD127* in stage (c) the CD4+CD25+FoxP3+CD127-CD26- phenotype is indicative of the presence of nTreg cells in the biological sample. When stage (a) of the second method of the invention detects the expression product of genes *CD4, CD26, CD25, FoxP3* and *CD49d* in stage (c) the CD4+CD25+FoxP3+CD49d-CD26- phenotype is indicative of the presence of nTreg cells in the biological sample. When stage (a) of the second method of the invention detects the expression product of genes *CD4, CD26, CD25, CD127* and *CD49d* in stage (c) the CD4+CD25+CD127-CD49d-CD26- phenotype is indicative of the presence of nTreg cells in the biological sample. When stage (a) of the second method of the invention detects the expression product of genes *CD4, CD26, FoxP3, CD127* and *CD49d* in stage (c) the CD4+FoxP3+CD127-CD49d-CD26-phenotype is indicative of the presence of nTreg cells in the biological sample. When stage (a) of the second method of the invention detects the expression product of genes *CD4, CD26, CD25, FoxP3, CD127* and *CD49d* in stage (c) the CD4+CD25+FoxP3+CD127-CD49d-CD26- phenotype is indicative of the presence of nTreg cells in the biological sample.

In a preferred embodiment, in stage (a) of the second method of the invention is also detected the amount of the expression product of at least one of the genes selected from the list comprising: TCRαβ, *CD3, CD5, CD11a*/*LFA-1, CD27, CD28, CD30, CD31, CD38, CD39, CD44, CD45RA, CD45RB, CD45RO, CD45RC, CD54*/*ICAM-1, CD57, CD58, CD62L*/*L-selectin, CD62P*/*P-selectin, CD71, CD73, CD83, CD80, CD86, CD95*/*Fas*/*APO-1, CD101, CD103, CD122*/*IL-2Rβ, CD132, CD134*/*OX-40, CD137*/*4-1BB, CD152*/*CTLA-4, CD154*/*CD40L, CD223*/*LAG - 3, PD - 1, PD - L1, GITR, IL - 10, TGFβ, galectin-1, Neuropilin*/*NRP, Neopterin, TNFR2, TGFβR1, G - protein - coupled receptor B3*/*GPRB3, HLA - DR, ICOS, GARP, FR4, TLR4, TLR5, TLR8, CRTH2, CCR7, CCR4, CCR8. CCR5, CXCR4, CXCR5, CLA, granzyme A and granzyme B.* Some of these genes are positive selection markers, others are negative selection markers and others allow differentiation of a specific population of nTreg cells.

Positive selection markers that can also be used in stage (a) of the second method of the invention are, for example, but without limitation, TCRαβ, *CD3, CD5, CD39, CD44, CD58, CD71, CD73, CD83, CD95*/*Fas*/*APO -* 1, *CD122*/*IL - 2Rβ, CD132, CD152*/*CTLA - 4, PD - 1, PD - L1, GITR, galectin -* 1, *Neuropilin*/*NRP, Neopterin, HLA - DR, ICOS, FR4* and *CLA.* In a preferred embodiment of the method of the invention in stage (a) is also detected the expression product of at least one of the genes selected from the list comprising: TCRαβ, *CD3, CD5, CD39, CD44, CD58, CD71, CD73, CD83, CD95*/*Fas*/*APO -* 1 *(*+*), CD122*/*IL - 2Rβ, CD132, CD152*/*CTLA - 4, PD - 1, PD - L1, GITR, galectin - 1, Neuropilin*/*NRP, Neopterin, HLA - DR, ICOS, FR4* and *CLA,* in such a way that in stage (c) identification of the nTreg cells is assigned when the amount detected of the expression product of any of those genes in stage (a) is significantly higher than the reference amount for said genes. Hence, the TCRαβ+, *CD3+, CD5+, CD39+, CD44*+*, CD58+, CD71+, CD73+, CD83+, CD95*/*Fas*/*APO-1+, CD122*/*IL-2Rβ+, CD132+, CD152*/*CTLA-4+, PD-1*+*, PD-L1*+*, GITR+, galectin-1+, Neuropilin*/*NRP+, Neopterin+, HLA-DR+, ICOS+, FR4+* and *CLA+* criteria can be incorporated into any of the phenotypes described above as indicative of nTreg cells.

Negative selection markers that can also be used in stage (a) of the second method of the invention are, for example, but without limitation, *CD57, CD137*/*4-1BB, CD154*/*CD40L, CRTH2* and *granzyme B.* Therefore, in a preferred embodiment of the method of the invention in stage (a) is also detected the expression product of at least one of the genes selected from the list comprising: *CD57, CD137*/*4-1BB, CD154*/*CD40L, CRTH2* and *granzyme B,* in such a way that in stage (c) identification of the nTreg cells is assigned when the amount detected of the expression product of any of those genes in stage (a) is significantly lower than the reference amount for said genes. Hence, the *CD57, CD137*/*4-1BB-, CD154*/*CD40L-, CRTH2-* and *granzyme B-* criteria can be incorporated into any of the phenotypes described above as indicative of nTreg cells.

Genes allowing differentiation of a specific population of nTreg cells that can also be used in stage (a) of the second method of the invention are, for example, but without limitation, *CD11a*/*LFA-1, CD27, CD30, CD31, CD38, CD45RA, CD45RB, CD45RO, CD45RC, CD54*/*ICAM-1, CD62L*/*L-selectin, CD62P*/*P-selectin, CD80, CD86, CD101, CD103, CD134*/*OX-40, CD223*/*LAG-3, IL-10, TGFβ, TNFR2, TGFβR1, G-protein-coupled receptor 83*/*GPR83, GARP, TLR4, TLR5, TLR8, CCR7, CCR4, CCR8, CCR5, CXCR4, CXCR5* and *granzyme A.* Therefore, in a preferred embodiment of the method of the invention in stage (a) is also detected the expression product of at least one of the genes selected from the list comprising: *CD11a*/*LFA-1, CD27, CD30, CD31, CD38, CD45RA, CD45RB, CD45RO, CD45RC, CD54*/*ICAM-1, CD62L*/*L-selectin, CD62P*/*P-selectin, CD80, CD86, CD101, CD103, CD134*/*OX-40, CD223*/*LAG-3, IL-10, TGFβ, TNFR2, TGFβR1, G-protein-coupled receptor 83*/*GPR83, GARP, TLR4, TLR5, TLR8, CCR7, CCR4, CCR8, CCR5, CXCR4, CXCR5* and *granzyme A.* Hence, in a preferred embodiment of the second method of the invention, in stage (a) is also detected the expression product of genes such as *CD11a*/*LFA-1, CD27, CD30, CD31, CD38, CD45RA, CD45RB, CD45RO, CD45RC, CD54*/*ICAM-1, CD62L*/*L-selectin, CD62P*/*P-selectin, CD80, CD86, CD101, CD103, CD134*/*OX-40, CD223*/*LAG-3, IL-10, TGFβ, TNFR2, TGFβR1, G-protein-coupled receptor 83*/*GPR83, GARP, TLR4, TLR5, TLR8, CCR7, CCR4, CCR8, CCR5, CXCR4, CXCR5* and *granzyme A,* in such a way as to identify in stage (c) a sub-population of nTreg cells *CD11a*/*LFA-1*+*, CD27+, CD30+, CD31*+*, CD38*+*, CD45RA*+*, CD45RB*+*, CD45RO*+*, CD45RC*+*, CD54*/*ICAM-1*+*, CD62L*/*L-selectin*+*, CD62P*/*P-selectin*+*, CD80*+*, CD86*+*, CD101+, CD103+, CD134*/*OX-40*+*, CD223*/*LAG-3*+*, IL-10*+*, TGFβ*+*, TNFR2+, TGFβR1+, G-protein-coupled receptor 83*/*GPR83+, GARP+, TLR4+, TLR5*+*, TLR8*+*, CCR7+, CCR4+, CCR8*+*, CCR5*+*, CXCR4*+*, CXCR5* + and *granzyme* A+ when the amount detected of their expression products in stage (a) is significantly higher than the reference amount.

The term "expression product", as used in the present description, refers to the products of transcription or expression (RNA or protein), or to any form resulting from the processing of such products of transcription or expression. In a preferred embodiment, the expression product of the genes detected in step (a) is mRNA. In a preferred embodiment, the expression product of the genes detected in step (a) is a protein.

The expression "detection of the expression product" of the genes in the biological sample, as used in the present description, refers to the measurement of the amount or concentration, preferably in a semi - quantitative or quantitative manner. The measurement can be carried out directly or indirectly. The expression "direct measurement of the amount of expression product" refers to the measurement of the amount or concentration of the expression product of the genes based on a signal that is obtained directly from the expression products of the genes and that is directly correlated to the number of molecules of the expression product of the genes present in the sample. Said signal may be obtained, for example, by measuring a value of the intensity of a chemical or physical property of the expression product. The expression "indirect measurement of the amount of expression product" refers to the measurement obtained from a secondary component (for example, a component other than the gene expression product) or biological measurement system (for example, but without limitation, the measurement of cell responses, ligands, labels, or enzyme reaction products).

According to the present invention, detection of the expression product of the genes can be carried out by any method for determining the amount of expression product. In a preferred embodiment, detection of the expression product of the genes is carried out by determining the level of mRNA resulting from its transcription, wherein the analysis of the level of mRNA can be carried out, for example, but without limitation, by means of polymerase chain reaction amplification (PCR), reverse transcriptase in combination with the polymerase chain reaction (RT - PCR), reverse transcriptase in combination with ligase chain reaction (RT - LCR), or any other nucleic acid amplification method; DNA *arrays* prepared with oligonucleotides deposited by any mechanism; DNA *arrays* prepared with synthetic oligonucleotides *in situ* by means of photolithography or any other mechanism; hybridisation *in situ* using specific probes marked with any marking method; by means of electrophoresis gels; by means of transfer to membrane and hybridisation with a specific probe; by means of nuclear magnetic resonance (NMR) or any other diagnostic imaging technique using paramagnetic nanoparticles or any other type of detectable nanoparticles functionalised with antibodies or by any other means.

In a more preferred embodiment, detection of the amount of expression product of the genes in the biological sample is carried out by determining the level of the proteins encoded by said genes, through incubation with a specific antibody, in tests such as *Western blot,* electrophoresis gels, immunoprecipitation, protein *arrays,* immunofluorescence, immunohistochemistry, ELISA or any other enzymatic method; by means of incubation with a specific ligand, for example adenosine deaminase, in the case of CD26; by means of NMR or any other diagnostic imaging technique; or, for example, by means of chromatography techniques combined with mass spectrometry.

Protein CD26 has dipeptidyl peptidase IV activity (DPPIV). Therefore, it is also possible to detect protein CD26 by analysing its dipeptidyl peptidase IV activity, as may be achieved for example by using the fluorogenic substrate [Ala - Pro]² - cresyl violet, in combination with confocal microscopy or flow cytometry.

The term "amount", as used in the description, refers but is not limited to, the absolute or relative amount of the expression product of the gene, as well as to any other value or parameter related thereto or that can be derived from it. Said values or parameters comprise values of the intensity of the signal obtained based on any of the physical or chemical properties of the expression products of the specified genes obtained by direct measurement, for example, intensity values of mass spectroscopy or nuclear magnetic resonance. Additionally, said values or parameters include all those obtained by indirect measurement, for example, using any of the measurement systems described elsewhere in this document.

The term "comparison", as used in the description, refers but is not limited to, the comparison of the amount of the expression product of one of the genes of the biological sample to be analysed with a desirable reference amount. The comparison described in section (b) of the method of the present invention can be carried out manually or computer - assisted.

The term "reference amount", as used in the description, refers to the amount of the expression product that allows identification of an nTreg cell to be assigned. In the case of certain experiments this reference amount can be determined through the use of control antibodies, with the same isotype and the same molecular labels as the specific antibodies used, which do not recognise any antigen within the sample under analysis. Said reference amount can also be determined using samples of total RNA or mRNA, or of protein mixtures or lysates, taken from tissues or cells in which the natural lack has been described of mRNA, protein, encoded or enzymatic activity associated to the gene in question. The reference amount may also be established by means of samples of total RNA or mRNA, or of protein mixtures or lysates, taken from tissues or cells in which the product or products associated to the gene in question have been removed, such as mRNA, protein or enzymatic activity, through means, without excluding others, such as nucleotidic probes or neutralising peptides or specific chemical inhibitors.

Preferably, the second method of the invention is a method for the identification of nTreg cells of an animal, more preferably nTreg cells of a mammal, and even more preferably, human nTreg cells.

A fifth aspect of the present invention relates to a kit (hereinafter, second kit of the invention), which comprises the elements necessary for the detection in a biological sample of the expression product of the *CD26* gene, and the expression product of at least one of the genes selected from the list that comprises: *CD25, FoxP3, CD127* and *CD49d.*

In a preferred embodiment, the second kit of the invention comprises the elements necessary for the detection in a biological sample of the expression product of the *CD26* gene, and the expression product of at least one of the genes selected from the list that comprises: *CD25, FoxP3, CD127* and *CD49d,* wherein said elements are selected from:
- a probe that allows detection of the expression product of a gene selected from the list comprising: *CD26, CD25, FoxP3, CD127* or *CD49d,* or
- a primer that allows detection of the expression product of a gene selected from the list comprising: *CD26, CD25, FoxP3, CD127* or *CD49d,* or
- an antibody that allows detection of a protein selected from the list comprising: CD26, CD25, FoxP3, CD127 or CD49d, or
- a reagent that allows detection of the dipeptidyl peptidase IV activity of the CD26 protein,
or any combination thereof.

In a more preferred embodiment, the second kit of the invention also comprises an antibody or a probe that allows detection of the expression product of the *CD4* gene. In an even more preferred embodiment, the second kit of the invention also comprises an antibody or a probe that allows detection of the expression product of at least one gene selected from the list comprising: *CD3, CD5, CD11a*/*LFA-1, CD27, CD38, CD45RA, CD45RB, CD45RO, CD54*/*ICAM-1, CD62L*/*L-selectin, CD62P*/*P-selectin, CD73, CD86*/*B7-2, CD95*/*Fas*/*APO-1, CD103, CD122*/*IL-2Rβ, CD134*/*OX-40, CD137*/*4-1BB, CD152*/*CTLA-4, CD154*/*CD40L, CD279*/*PD-1, GITR, IL-10, TGFβ, galectin-1, Neuropilin*/*NRP, TNFR2, TGFβR1, G-protein-coupled receptor 83*/*GPR83* and *HLA-DR.*

The term "probe", as used in the present description refers to a molecule of single-stranded RNA or DNA with a specific base sequence, preferably marked, which is used to detect complementary base sequences to the expression product of a gene through partial or total hybridisation. Probes can be, for example, but without limitation, an oligonucleotide, a fragment amplified by PCR or a purified DNA fragment. The expression "probe of the expression product of a gene" refers to a probe that allows detection of the expression of said gene, in other words, refers to a probe complementary to the mRNA or cDNA of said gene.

In a preferred embodiment, the second kit of the invention comprises a probe of the expression product of the *CD26* gene and a probe of the expression product of a gene selected from the list comprising: *CD25, FoxP3, CD127* or CD49d. In a more preferred embodiment, the second kit of the invention comprises a probe of the expression product of the *CD26* gene and a probe of the expression product of the *CD25* gene. In a more preferred embodiment, the second kit of the invention comprises a probe of the expression product of the *CD26* gene and a probe of the expression product of the *FoxP3* gene. In another more preferred embodiment, the second kit of the invention comprises a probe of the expression product of the *CD26* gene and a probe of the expression product of the *CD127* gene. In another more preferred embodiment, the second kit of the invention comprises a probe of the expression product of the *CD26* gene and a probe of the expression product of the *CD49d* gene. In another more preferred embodiment, the second kit of the invention comprises a probe of the expression product of the *CD26* gene, a probe of the expression product of the *CD25* gene and a probe of the expression product of the *FoxP3* gene. In another more preferred embodiment, the second kit of the invention comprises a probe of the expression product of the *CD26* gene, a probe of the expression product of the *CD25* gene and a probe of the expression product of the *CD127* gene. In another more preferred embodiment, the second kit of the invention comprises a probe of the expression product of the *CD26* gene, a probe of the expression product of the *FoxP3* gene and a probe of the expression product of the *CD127* gene. In another more preferred embodiment, the second kit of the invention comprises a probe of the expression product of the *CD26* gene, a probe of the expression product of the *CD25* gene and a probe of the expression product of the *CD49d* gene. In another more preferred embodiment, the second kit of the invention comprises a probe of the expression product of the *CD26* gene, a probe of the expression product of the *FoxP3* gene and a probe of the expression product of the *CD49d* gene. In another more preferred embodiment, the second kit of the invention comprises a probe of the expression product of the *CD26* gene, a probe of the expression product of the *CD127* gene and a probe of the expression product of the *CD49d gene.* In another more preferred embodiment, the second kit of the invention comprises a probe of the expression product of the *CD26* gene, a probe of the expression product of the *CD25* gene, a probe of the expression product of the *FoxP3* gene and a probe of the expression product of the *CD127* gene. In another more preferred embodiment, the second kit of the invention comprises a probe of the expression product of the *CD26* gene, a probe of the expression product of the *CD25* gene, a probe of the expression product of the *FoxP3* gene and a probe of the expression product of the *CD49d* gene. In another more preferred embodiment, the second kit of the invention comprises a probe of the expression product of the *CD26* gene, a probe of the expression product of the *FoxP3* gene and a probe of the expression product of the *CD127 gene.* In another more preferred embodiment, the second kit of the invention comprises a probe of the expression product of the *CD26* gene, a probe of the expression product of the *CD25* gene, a probe of the expression product of the *CD127* gene and a probe of the expression product of the *CD49d gene.* In another more preferred embodiment, the second kit of the invention comprises a probe of the expression product of the *CD26* gene, a probe of the expression product of the *FoxP3* gene, a probe of the expression product of the *CD127* gene and a probe of the expression product of the *CD49d* gene. In another more preferred embodiment, the second kit of the invention comprises a probe of the expression product of the *CD26* gene, a probe of the expression product of the *CD25* gene, a probe of the expression product of the *FoxP3* gene, a probe of the expression product of the *CD127* gene and a probe of the expression product of the *CD49d* gene. In an even more preferred embodiment, the second kit of the invention additionally comprises a probe of the expression product of the CD4 gene. In an even more preferred embodiment, the second kit of the invention comprises also a probe of the expression product of a gene selected from the list comprising: TCRαβ, *CD3, CD5, CD11a*/*LFA-1, CD27, CD28, CD30, CD31, CD38, CD39, CD44, CD45RA, CD45RB, CD45RO, CD45RC, CD54*/*ICAM-1, CD57, CD58, CD62L*/*L-selectin, CD62P*/*P-selectin, CD71, CD73, CD83, CD80, CD86, CD95*/*Fas*/*APO-1, CD101, CD103, CD122*/*IL-2Rβ, CD132, CD134*/*OX-40, CD137*/*4-1BB, CD152*/*CTLA-4, CD154*/*CD40L, CD223*/*LAG-3, PD-1, PD-L1, GITR, IL-10, TGFβ, galectin-1, Neuropilin*/*NRP, Neopterin, TNFR2, TGFβR1, G-protein-coupled receptor 83*/*GPR83, HLA-DR, ICOS, GARP, FR4, TLR4, TLR5, TLR8, CRTH2, CCR7, CCR4, CCR8, CCR5, CXCR4, CXCR5, CLA, granzyme A and granzyme B.*

The term "primer", as used herein, refers to an oligonucleotide of DNA or RNA complementary to the sequence of a specific template nucleic acid, which acts as the starting point for adding nucleotides in the process of copying the chain complementary to the sequence of the aforesaid template nucleic acid, for example, but without limitation, in a PCR. Preferably, the primer is an oligonucleotide of DNA. The expression "primer of the expression product of the gene" refers to a primer that allows detection of the expression of said gene, in other words, it refers to a primer that is complementary to the mRNA or cDNA of said gene. Primers that can be used in the invention are known in the state of the art or can be easily designed on the basis of the gene sequences, for example, based on the sequence of the genes *CD26, CD25, FoxP3, CD127, CD49d* or *CD4.*

In a preferred embodiment, the second kit of the invention comprises at least one primer of the expression product of the *CD26* gene and one primer of the expression product of a gene selected from the list comprising: *CD25, FoxP3, CD127* or CD49d. In a more preferred embodiment, the second kit of the invention comprises at least one primer of the expression product of the *CD26* gene and at least one primer of the expression product of the *CD25* gene. In a more preferred embodiment, the second kit of the invention comprises at least one primer of the expression product of the *CD26* gene and at least one primer of the expression product of the *FoxP3* gene. In another more preferred embodiment, the second kit of the invention comprises at least one primer of the expression product of the *CD26* gene and at least one primer of the expression product of the *CD127* gene. In another more preferred embodiment, the second kit of the invention comprises at least one primer of the expression product of the *CD26* gene and at least one primer of the expression product of the *CD49d gene.* In another more preferred embodiment, the second kit of the invention comprises at least one primer of the expression product of the *CD26* gene, at least one primer of the expression product of the *CD25* gene and at least one primer of the expression product of the *FoxP3* gene. In another more preferred embodiment, the second kit of the invention comprises at least one primer of the expression product of the *CD26* gene, at least one primer of the expression product of the *CD25* gene and at least one primer of the expression product of the *CD127* gene. In another more preferred embodiment, the second kit of the invention comprises at least one primer of the expression product of the *CD26* gene, at least one primer of the expression product of the *FoxP3* gene and at least one primer of the expression product of the *CD127* gene. In another more preferred embodiment, the second kit of the invention comprises at least one primer of the expression product of the *CD26* gene, at least one primer of the expression product of the *CD25* gene and at least one primer of the expression product of the *CD49d* gene. In another more preferred embodiment, the second kit of the invention comprises at least one primer of the expression product of the *CD26* gene, at least one primer of the expression product of the *FoxP3* gene and at least one primer of the expression product of the *CD49d* gene. In another more preferred embodiment, the second kit of the invention comprises at least one primer of the expression product of the *CD26* gene, at least one primer of the expression product of the *CD127* gene and at least one primer of the expression product of the *CD49d* gene. In another more preferred embodiment, the second kit of the invention comprises at least one primer of the expression product of the *CD26* gene, at least one primer of the expression product of the *CD25* gene, at least one primer of the expression product of the *FoxP3* gene and at least one primer of the expression product of the *CD127* gene. In another more preferred embodiment, the second kit of the invention comprises at least one primer of the expression product of the *CD26* gene, at least one primer of the expression product of the *CD25* gene, at least one primer of the expression product of the *FoxP3* gene and at least one primer of the expression product of the *CD49d gene.* In another more preferred embodiment, the second kit of the invention comprises at least one primer of the expression product of the *CD26* gene, at least one primer of the expression product of the *FoxP3* gene and at least one primer of the expression product of the *CD127* gene. In another more preferred embodiment, the second kit of the invention comprises at least one primer of the expression product of the *CD26* gene, at least one primer of the expression product of the *CD25* gene, at least one primer of the expression product of the *CD127* gene and at least one primer of the expression product of the *CD49d gene.* In another more preferred embodiment, the second kit of the invention comprises at least one primer of the expression product of the *CD26* gene, at least one primer of the expression product of the *FoxP3* gene, at least one primer of the expression product of the *CD127* gene and at least one primer of the expression product of the *CD49d* gene. In another more preferred embodiment, the second kit of the invention comprises at least one primer of the expression product of the *CD26* gene, at least one primer of the expression product of the *CD25* gene, at least one primer of the expression product of the *FoxP3* gene, at least one primer of the expression product of the *CD127* gene and at least one primer of the expression product of the *CD49d gene.* In an even more preferred embodiment, the second kit of the invention also comprises at least one primer of the expression product of the CD4 gene. In an even more preferred embodiment, the second kit of the invention also comprises at least one primer of the expression product of a gene selected from the list comprising: TCRαβ, *CD3, CD5, CD11a*/*LFA-1, CD27, CD28, CD30, CD31, CD38, CD39, CD44, CD45RA, CD45RB, CD45RO, CD45RC, CD54*/*ICAM-1, CD57, CD58, CD62L*/*L-selectin, CD62P*/*P-selectin, CD71, CD73, CD83, CD80, CD86, CD95*/*Fas*/*APO-1, CD101, CD103, CD122*/*IL-2Rβ, CD132, CD134*/*OX-40, CD137*/*4-1BB, CD152*/*CTLA-4, CD154*/*CD40L, CD223*/*LAG-3, PD-1, PD-L1, GITR, IL-10, TGFβ, galectin-1, Neuropilin*/*NRP, Neopterin, TNFR2, TGFβR1, G-protein-coupled receptor 83*/*GPR83, HLA-DR, ICOS, GARP, FR4, TLR4, TLR5, TLR8, CRTH2, CCR7, CCR4, CCR8, CCR5, CXCR4, CXCR5, CLA, granzyme A* and *granzyme B.*

In a preferred embodiment, the second kit of the invention comprises at least one anti-CD26 antibody and an antibody selected from the list comprising: an anti-CD25 antibody, anti-FoxP3 antibody, anti-CD127 or anti-CD49d antibody. In a more preferred embodiment, the second kit of the invention comprises an anti-CD26 antibody and an anti-CD25 antibody. In a more preferred embodiment, the second kit of the invention comprises an anti-CD26 antibody and an anti-FoxP3 antibody. In another more preferred embodiment, the second kit of the invention comprises an anti-CD26 antibody and an anti-CD127 antibody. In another more preferred embodiment, the second kit of the invention comprises an anti-CD26 antibody and an anti-CD49d antibody. In another more preferred embodiment, the second kit of the invention comprises an anti-CD26 antibody, and anti-CD25 antibody and an anti-FoxP3 antibody. In another more preferred embodiment, the second kit of the invention comprises an anti-CD26 antibody, an anti-CD25 antibody and an anti-CD127 antibody. In another more preferred embodiment, the second kit of the invention comprises an anti-CD26 antibody, an anti-FoxP3 antibody and an anti-CD127 antibody. In another more preferred embodiment, the second kit of the invention comprises an anti-CD26 antibody, an anti-CD25 antibody and an anti-CD49d antibody. In another more preferred embodiment, the second kit of the invention comprises an anti-CD26 antibody, an anti-FoxP3 antibody and an anti-CD49d antibody. In another more preferred embodiment, the second kit of the invention comprises an anti-CD26 antibody, an anti-CD127 antibody and an anti-CD49d antibody. In another more preferred embodiment, the second kit of the invention comprises an anti-CD26 antibody, an anti-CD25 antibody, an anti-FoxP3 antibody and an anti-CD127 antibody. In another more preferred embodiment, the second kit of the invention comprises an anti-CD26 antibody, an anti-CD25 antibody, an anti-FoxP3 antibody and an anti-CD49d antibody. In another more preferred embodiment, the second kit of the invention comprises an anti-CD26 antibody, an anti-FoxP3 antibody and an anti-CD127 antibody. In another more preferred embodiment, the second kit of the invention comprises an anti-CD26 antibody, an anti-CD25 antibody, an anti-CD127 antibody and an anti-CD49d antibody. In another more preferred embodiment, the second kit of the invention comprises an anti-CD26 antibody, an anti-FoxP3 antibody, an anti-CD127 antibody and an anti-CD49d antibody. In another more preferred embodiment, the second kit of the invention comprises an anti-CD26 antibody, an anti-CD25 antibody, an anti-FoxP3 antibody, an anti-CD127 antibody and an anti-CD49d antibody. In an even more preferred embodiment, the second kit of the invention also comprises an anti-CD4 antibody. In an even more preferred embodiment, the second kit of the invention also comprises an antibody selected from the list comprising: anti-CD3 antibody, anti-CD5 antibody, anti-CD11a/LFA-1 antibody, anti-CD27 antibody, anti-CD38 antibody, anti-CD45RA antibody, anti-CD45RB antibody, anti-CD45RO antibody, anti-CD54/ICAM-1 antibody, anti-CD62L/L-selectin antibody, anti-CD62P/P-selectin antibody, anti-CD73 antibody, anti-CD86/B7- 2 antibody, anti-CD95/Fas/APO-1 antibody, anti-CD103 antibody, anti-CD122/IL-2Rβ antibody, anti-CD134/OX-40 antibody, anti-CD137/4-1BB antibody, anti-CD152/CTLA-4 antibody, anti-CD154/CD40L antibody, anti-CD279/PD-1 antibody, anti-GITR antibody, anti-IL-10 antibody, anti-TGFβ antibody, anti-galectin-1 antibody, anti-Neuropilin/NRP antibody, anti-TNFR2 antibody, anti-TGFβR1 antibody, anti-G-protein-coupled receptor 83/GPR83 antibody and anti-HLA-DR antibody.

In a preferred embodiment, at least one of the antibodies of the second kit of the invention is marked or immobilised. In a preferred embodiment, at least one of the antibodies of the second kit of the invention is marked with a label selected from the list comprising: a radioisotope, a fluorescent or luminescent marker, an antibody, an antibody fragment, an affinity label, an enzyme and an enzyme substrate. In a preferred embodiment, at least one of the antibodies of the second kit of the invention is immobilised in a nylon matrix, in a plastic support or in beads.

Taking into account that it is possible to detect CD26 protein by analysing its dipeptidyl peptidase IV activity, in a preferred embodiment, the second kit of the invention also comprises at least one reagent that allows detection of the dipeptidyl peptidase IV activity of the CD26 protein. The expression "reagent that allows detection of the dipeptidyl peptidase IV activity of the CD26 protein", as used in the present description, refers to a molecule through which the enzyme is very specific (for example, the Ala - Pro dipeptide in the case of CD26/DPP-IV) and the processing of which generates a signal (fluorogenic, chromogenic, etc.) of intensity directly proportional to the specific activity (amount) of the enzyme, or alternatively that presents physical - chemical properties (for example, a new length of emission wave) that allow differentiation from the signal coming from the unprocessed molecule. Examples of reagents that can be used to detect the dipeptidyl peptidase IV activity are, for example, and without limitation, the chromogenic substrates glycyl-L-prolyl-p-nitroanilide (Gly-Pro-pNA) or glycyl-L-prolyl-3,5-dibromo-4-hydroxyanilide (Gly-Pro-DBAP) (both combined with spectrophotometers), or the fluorogenic substrate [Ala - Pro]² - cresyl violet (combined with confocal microscopy or flow cytometry).

The second kit of the invention may also contain any other reagent necessary for executing the method of the invention such as for example, but without limitation, positive and/or negative controls, buffers, anti - contamination agents, protein degradation inhibitors, polymerases, nucleotides, etc. Additionally, the second kit of the invention may include any support and/or container necessary for start up and optimisation. Preferably, the second kit of the invention also comprises the instructions for carrying out the method of the invention.

A sixth aspect of the present invention relates to the use of the second kit of the invention for the identification of nTreg cells from an isolated biological sample. Preferably, the biological sample is taken from an individual having an autoimmune disease, an allergic disease, an inflammatory disease, an infectious disease or a parasitic disease.

Throughout this description and the claims the word "comprises" and its variants do not intend to exclude other technical characteristics, additives, components or steps. For persons skilled in the art, other purposes, advantages and characteristics of the invention shall be inferred in part from the description and in part from the practice of the invention. The following figures and examples are provided by way of illustration, and are not intended to limit the present invention.

### DESCRIPTION OF THE DRAWINGS

**Figure 1****. Diagram showing a potential application of the use of CD26 in a protocol for the magnetic purification of human nTreg cells through negative selection.**

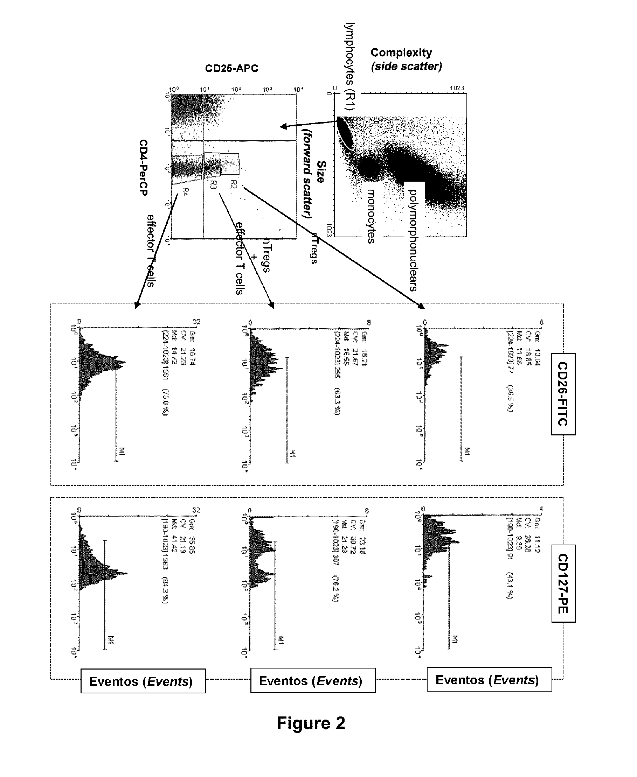

**Figure 2****. Shows how human naturally - occurring T regulatory cells (nTreg) present low or no expression of CD26.** The blood was marked directly with specific monoclonal antibodies of CD4, CD25, CD26 (clone TP1/19) and CD127, subsequently lysing the erythrocytes, fixing the leukocytes and reading the samples in the cytometer. In the subsequent analysis, the population of CD4+ lymphocytes was selected according to cell size and complexity (R1; forward scatter of light vs. side scatter of light, respectively). Against this basis, and based on the expression of CD4 and CD25, 3 sub-populations were established (R2 - R4); measuring the extracellular expression of CD26 and CD127 in each one of them.

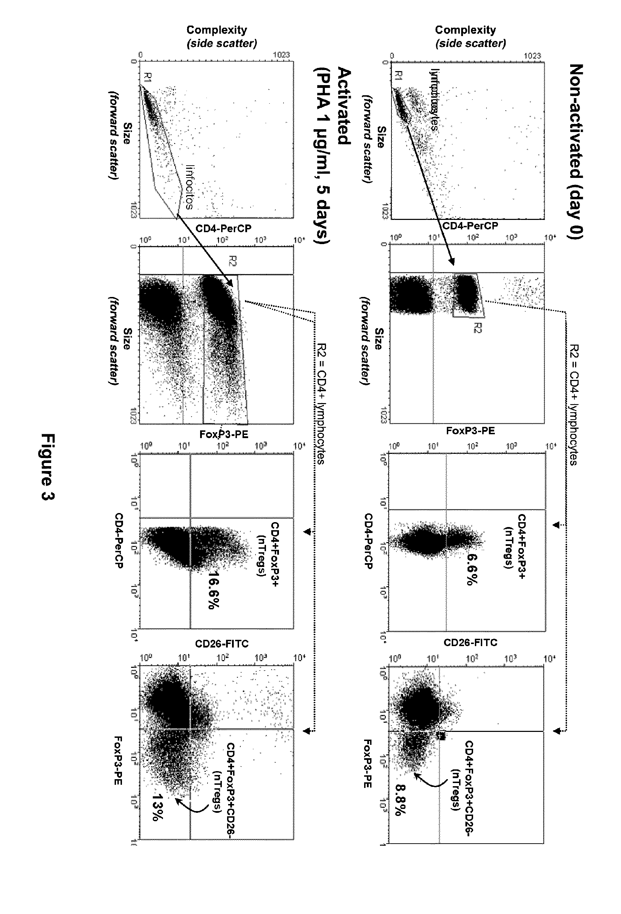

**Figure 3****. Shows how use of the CD4+FoxP3+ phenotype is insufficient for determining human nTregs in activated T cell populations.** PBMCs were purified first by density gradient in Ficoll, and either marked directly (non - activated, day 0), or activated during 5 days in complete medium supplemented with 1 µg/ml PHA before proceeding with marking with antibodies. In both cases, the cells were initially incubated with anti-CD26 (clone TP1/16) and anti-CD4, both extracellular markers, before being fixed/permeabilised and marked with the anti-FoxP3 antibody. During data analysis, first lymphocytes were selected according to cell size/complexity (R1; forward scatter of light vs. side scatter of light, respectively), and within these CD4+ (R2). Subsequently, nTregs were identified using the FoxP3 marker (graph FoxP3-PE vs. CD4-PerCP; 6.6% nTreg in non-activated populations vs. 16.6% nTreg in activated) or in combination with CD26 (graph FoxP3-PE vs. CD26-FITC; 8.8% nTreg in non-activated vs. 13% nTreg in activated).

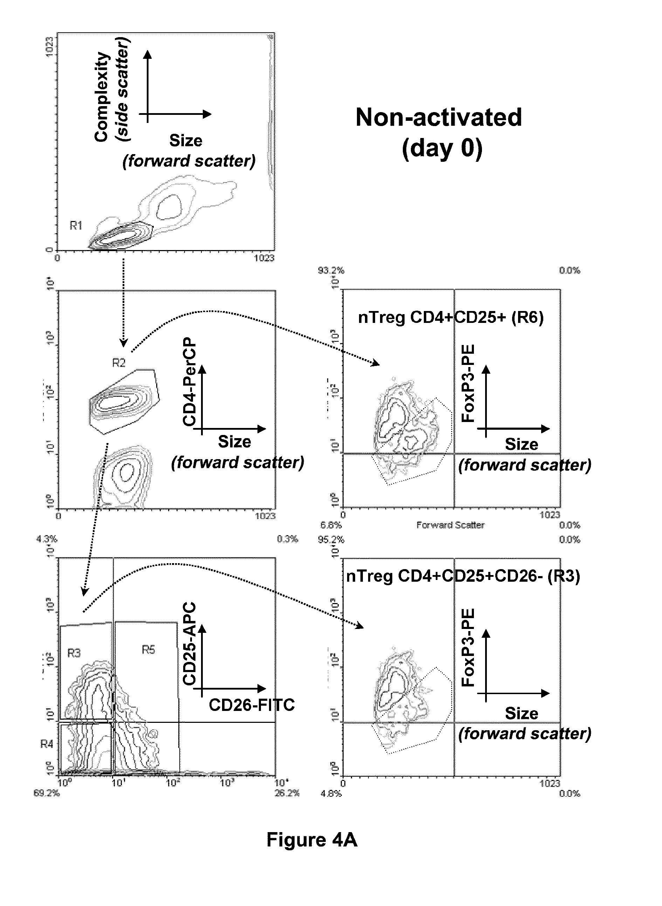

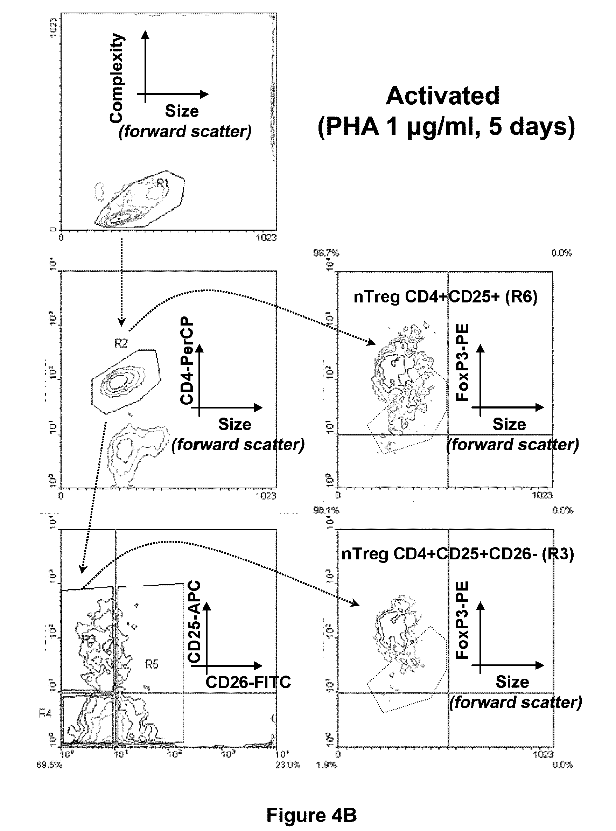

**Figure 4****. Proves how inclusion of the CD26 marker in the panel of CD4 and CD25 markers during the selection/purification process of human nTreg lymphocytes eliminates ambiguity in their identification as a result of using the CD25 marker.** As in Figure 3, PBMCs were purified and marked directly (Figure 4, part A: non-activated, day 0) or activated with PHA (Figure 4, part B: activated, 5 days) before immunofluorescent marking. During the latter, cells were incubated with anti-CD4-PerCP, anti-CD25-APC and anti-CD26-FITC (clone TP1/16) antibodies. Before marking with anti-FoxP3-PE, the lymphocytes were previously fixed and permeabilised. During analysis the population of lymphocytes was selected on the basis of size and complexity (R1), and within these the CD4+ cells (R2). In order to measure the percentage of FoxP3+ cells (graph FoxP3-PE vs. size) first those lymphocytes with phenotype CD25+ (R6) o CD25+CD26- (R3) were selected (graph CD26-FITC vs. CD25-APC). In non-activated PBMCs **(****Figure 4****, part A)** as well as in activated ones **(****Figure 4****, part B)** the CD4+CD25+ (R6) population presented a group of effector T cells with low FoxP3 levels (R7), non-existent within CD4+CD25+CD26- lymphocytes (R3).

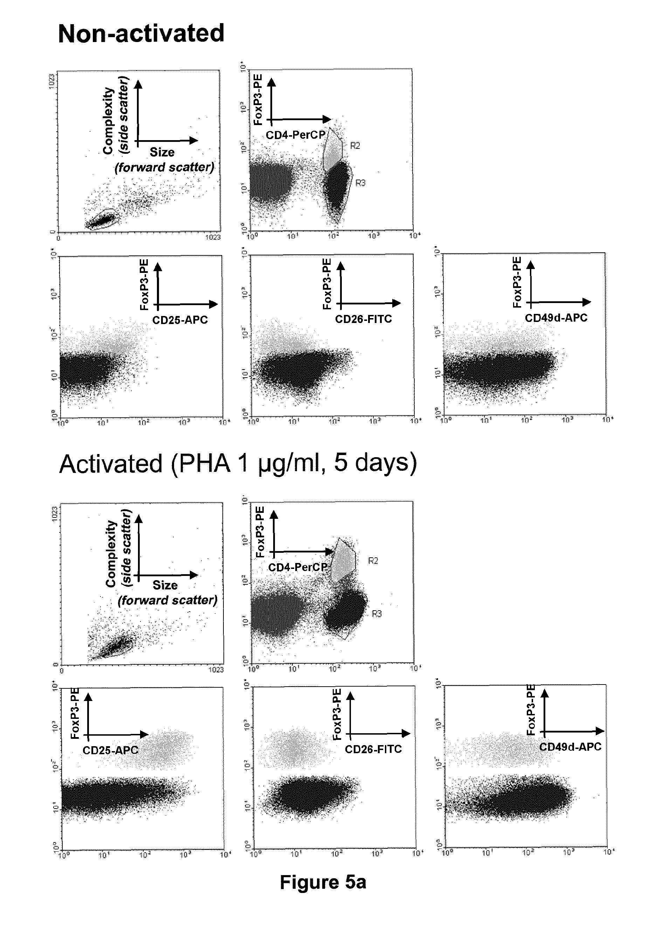

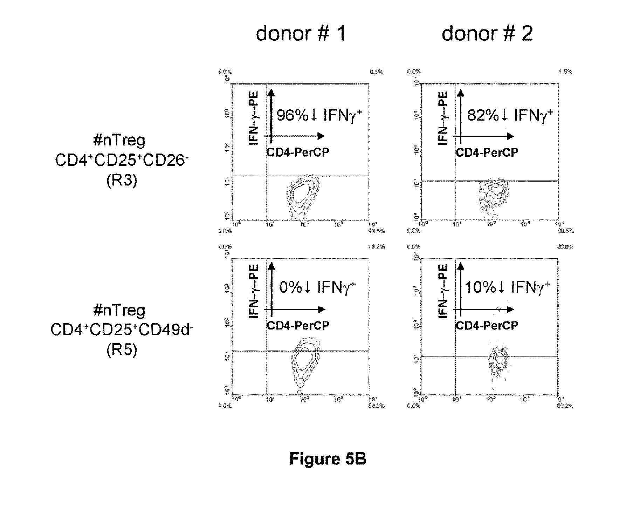

**Figure 5****. Proves how CD26, in comparison with CD49d, presents more notable differences in expression between nTreg lymphocytes (FoxP3⁺) and effector lymphocytes (FoxP3^{weakI/-}), and is also a better marker of IFNγ producer cells.** In **Figure 5****, part A,** PBMCs were purified and marked directly (non-activated, day 0) or activated with PHA during 5 days (activated) before immunofluorescent marking. Lymphocytes were incubated with anti-CD4-PerCP, anti-CD26-FITC (TP1/16) antibodies and, alternatively, anti-CD25-APC or anti-CD49d-APC. Subsequently, they were fixed and permeabilised before marking with anti-FoxP3-PE. During analysis, the population of lymphocytes was selected (size vs. complexity; R1), distinguishing two sub-populations within these based on CD4 and FoxP3: CD4+FoxP3+ nTregs (R2) and CD4⁺FoxP3^{weak**/**-}effector lymphocytes (R3). Finally, expression of CD25, CD26 and CD49d was analysed, in combination with FoxP3, within each one of these sub-populations (R2 and R3). For ease of comparison, nTreg lymphocytes have been shown in grey and effector cells in black. In **Figure 5****, part B,** PBMCs were purified, activated during 5 days with PHA and treated during 4 hours with BD GolgiStop™. Lymphocytes were incubated with two combinations of antibodies: anti-CD4-PerCP, anti-CD25-APC and anti-CD26-FITC (clone TP1/16), or anti-CD4-PerCP, anti-CD25-FITC and anti-CD49d-APC. Subsequently they were washed, fixed and permeabilised before marking with anti-IFNγ-PE. During data collection and analysis the population of lymphocytes was selected (size vs. complexity; R1; not shown), and within these CD4⁺CD25⁺ nTregs (R2; not shown). The bottom figures (CD4-PerCP vs. IFNγ-PE) show, for two healthy donors (#1 and #2) the amount of IFNγ + cells within nTreg cells CD4⁺CD25⁺CD26⁻ (R3; 0.5% and 1.5%) or CD4⁺CD25⁺CD49d⁻ (R5; 19.2% and 30.8%), as well as the reduction percentages of IFNγ⁺ cells in respect of the initial population CD4⁺CD25⁺ (R2) when CD26⁺(96%↓ and 82%1) or CD49d⁺(0%↓ and 10%↓) cells are removed.

### EXAMPLES

The following specific examples provided in this patent document serve to illustrate the nature of the present invention. These examples are included for illustrative purposes only and must not be interpreted as limitations of the invention claimed herein. Therefore, the examples described below illustrate the invention without limiting the field of application thereof.

### EXAMPLE 1. Identification of human nTreg cells using the CD26 marker.

### MATERIALS AND METHODS.

### 1. Materials.

Phytohemagglutinin (PHA), paraformaldehyde (PFA), penicillin-streptomycin solution and the culture medium RPMI-1640 were obtained from Sigma, foetal bovine serum (FBS) from Biowhittaker (Lonza) and Ficoll - Paque PLUS from GE Healthcare. Mouse antibodies against human molecules CD127 (anti-CD127-PE, IgG1, clone hIL-7R-M21), CD25 (anti-CD25-FITC, and anti-CD25-APC, both IgG1, clone M-A251), CD49d (anti-CD49d-APC, IgG1, clone 9F10), FoxP3 (anti - FoxP3 - PE, IgG1, clone 259D/C7) and IFNγ (anti - IFNγ-PE, IgG1, clone B27), as well as mouse isotypic control IgG1 - APC (clone X40), are from BD Pharmingen (BD Biosciences). Mouse antibodies against human molecules CD4 (CD4 - PerCP, IgG1, clone SK3) and CD26 (CD26 - FITC, IgG2a, clone L272) are from BD Immunocytometry Systems (BD Biosciences). The mouse antibody against human CD26, clone TP1/16, was purified in our own laboratory from a hybridoma supernatant and subsequently marked with FITC (*Fluorotag FITC Conjugation Kit,* Sigma). The murine antibody against human CD26 (CD26-FITC, IgG2b, clone TP1/19) was provided by lmmunostep (Salamanca, Spain). Isotypes IgG1 - FITC (clone MOPC - 21) and IgG1 - PE (clone MOPC - 21) were bought from Sigma. The buffers used for intracellular staining of FoxP3 and IFNγ were *BD Pharmingen stain buffer, BD FACS 10x lysing buffer* and *Human FoxP3 buffer set,* BD Cytofix/Cytoperm *buffer,* and *BD Perm*/*Wash™,* all from BD Biosciences.

### 2. Methods.

### PURIFICATION AND CULTURE OF PBMCs.

Peripheral blood mononuclear cells (PBMCs) were obtained from leukocyte concentrates from the Transfusion Centre of Galicia (Santiago de Compostela) through purification in Ficoll® gradients and used immediately (PBMCs) or cultivated at 37°C in an atmosphere with 5% CO2 in the presence of 1 µg/ml PHA (phytohemagglutinin of *Phaseolus vulgaris*) during 5 days in order to transform them into activated PBMCs (also known as blasts or lymphoblasts); in measurement tests of the percentage of IFNγ producer cells (Figure 5, part B), the culture medium was supplemented with a 1/1000 dilution of *GolgiPlug™* (BD Biosciences) during the last 4 hours of culture. In all experiments, the culture medium employed was RPMI-1640, supplemented with 10% foetal bovine serum (FBS), 100 µg/ml of streptomycin and 100 Ul/ml of penicillin, and the starting cell concentration was always 1x10⁶ PBMC/ml.

### IMMUNOFLUORESCENT LABELLING OF EXTRACELLULAR MARKERS IN UNFRACTIONATED BLOOD LYMPHOCYTES

For these experiments (Figure 2, unfractionated leukocytes), first the corresponding amount of antibody was added per tube: isotype - FITC, isotype - PE or isotype - APC (separately) for negative marking controls, and anti - CD26 - FITC (TP1/19), anti - CD127 - PE, anti - CD4 - PerCP or anti - CD25 - APC (all together for phenotyping or separately for compensations). The volume of each antibody used per tube was as specified by the manufacturer. Next, 100 µl of complete anticoagulated blood was deposited (K3E tubes, BD vacutainer), mixed well with the antibodies and incubated for 30 minutes in the dark at room temperature (RT). After this time, the erythrocytes were lysed adding 2 ml per tube of *1X BD FACS lysing solution* and incubating in the dark at RT during 15 minutes. Upon completion of the latter, the samples were centrifuged at 200 xg (5 min, RT) and the supernatant was removed. Next, the cells from the tube were washed in 2 ml of solution (PBS pH 7.4, 1 % foetal bovine serum /FBS, 0.1% sodium azide), repeating centrifugation at 200 xg (5 min, RT). After eliminating the supernatant, cells were fixed (1 ml of 2% paraformaldehyde/PFA in PBS pH 7.4, cold) during 30 min at RT. To stop fixation, leukocytes were washed with 2 ml per tube of wash buffer, centrifuging at 200 xg (5 min, RT), removing the supernatant and resuspending in 1 ml of wash buffer.

### IMMUNOFLUORESCENT MARKING OF EXTRACELLULAR AND INTRACELLULAR MARKERS IN PBMCs AND BLASTS.

For the simultaneous detection by immunofluorescence of both extracellular proteins (CD4, CD25, CD26, CD49d) as well as intracellular proteins (FoxP3) the following protocol was performed [Figures 3, 4 (part A and B) and 5 (part A)]. Both PBMCs as well as blasts (PHA 1 µg/ml, 5 days) were washed using *BD Pharmingen Stain Buffer,* counted using a haemocytometer and adjusted to a concentration of 10x10⁶ cells/ml with the same buffer. For extracellular marking appropriate volumes were placed in the cytometry tubes (following the manufacturer's instructions) of the isotype - FITC or isotype - APC (extracellular negative controls) or of the specific antibodies (anti - CD26 - FITC TP1/16, anti - CD4 - PerCP, anti - CD25 - APC, anti - CD49d - APC); all together for phenotyping, or separately for compensations). Next, 1x10⁶ cells (100 µl) were placed inside each cytometry tube and marking took place during 20 min at RT and in the dark. After washing with 2 ml of *BD Pharmingen Stain Buffer* per tube, cells were centrifuged at 250 xg 10 min RT. Supernatant was removed and cells were fixed with 2 ml per tube of *1x Human FoxP3 buffer A* during 10 min at RT in the dark. Following centrifugation at 500 xg 5 min RT, excess liquid was removed and the wash and centrifuging process repeated once more. Cells fixed in this way were permeabilised with 0.5 ml/tube of *1x Human FoxP3 buffer C* (1: 50 dilution of *Human FoxP3 buffer B in 1x Human FoxP3 buffer A)* during 30 min at RT in the dark. Cells were washed in 2 ml of BD *Pharmingen Stain Buffer* and centrifuged at 500 xg (5 min, RT). Supernatant was removed and the wash process repeated. After permeabilising the plasmatic membrane, cells were incubated during 30 minutes (RT, dark) either with the isotype - PE (intracellular isotope control; separately) or with anti - FoxP3 - PE (separately, for compensation, or in combination with the antibodies - already bound to the cells - anti - CD26 - FITC, anti - CD4 - PerCP, anti - CD25 - APC and anti - CD49d - APC for phenotyping). Cells were washed with 2 ml of *BD Pharmingen Stain Buffer,* centrifuged at 500 xg (5 min, RT) and fixed in 1 ml of 2 % PFA for 30 min at RT (dark). Following a last wash with 2 ml of *BD Pharmingen Stain Buffer,* leukocytes were resuspended in 1 ml of the same buffer.

In order to evaluate the percentage of IFNγ producer lymphoblasts (Figure 5, part B) a commercial protocol was also used (*BD Cytofix*/*Cytoperm™ PLUS Fixation*/*Permeabilization kit with BD GolgiPlug™*) very similar to the previous one, in which the cells were marked extracellularly with diverse combinations of the isotopic antibodies (isotype - FITC, isotype - APC) or specific antibodies (anti - CD26 - FITC TP1/16, anti - CD25 - FITC, anti - CD4 - PerCP, anti - CD49d - APC and anti - CD25 - APC). After washing twice with 1 ml of *BD Pharmingen Stain Buffer,* the cells were fixed and permeabilised during 20 minutes at 4 °C with 250 µl of BD Cytofix/Cytoperm *buffer,* and washed twice with 1 ml of *1x BD Perm*/*Wash™.* Incubation with anti - IFNγ - PE (or with isotype - PE as negative control) was carried out in 50 µl of *1x BD Perm*/*Wash™* during 30 minutes at 4 °C. Finally, cells were washed twice with 1 ml of *1x BD Perm*/*Wash™* and resuspended in 1 ml of *BD Pharmingen Stain Buffer.*

All cytometry data was collected in a FACScalibur cytometer (BD Biosciences), and analysed using WinMDI software, a free software package provided by Dr Joe Trotter (The Scripps Institute, Flow Cytometry Core Facility)

### RESULTS.

Figure 2 (unfractionated and non - activated leukocytes) shows how nTreg cells express low levels of CD26 in blood taken from healthy donors, CD4+ T lymphocytes with higher levels of CD25 (R2), and therefore nTreg cells, are those that have lower amounts of CD127 and CD26 on the cell surface. However, higher levels of CD127 and CD26 correspond to effector cells CD4+CD25- (R4); the population of CD4+ cells with intermediate levels of CD25 (R3) comprises both nTreg cells (CD26- and CD127-) as well as effector T cells (CD26+ and CD127+). These same results have been obtained using three different anti - CD26 monoclonal antibodies (TP1/19, Figure 2; TP1/16, Figure 5, parte A; L272, not shown), which points to the fact that it is not a specific epitope, but rather that there is a real reduction of the CD26 molecule on the surface of the nTreg cells.

In resting lymphocytes, 5.3 ± 2.2% (n = 10) of all non - activated CD4+ cells are CD25+. In the murine system, 100% of these CD25+ cells would be considered nTreg cells, but this assumption is incorrect in the human system, given that only those with very high levels of CD25 (population R2, but not R3, in Figure 2) can be considered true nTreg cells (100% FoxP3+); therefore, the percentage of nTreg cells with phenotype CD25+FoxP3+ within the CD4+ is even lower than 5%. Within these CD4+CD25+ nTregs, our data indicates that 81.9±11.2% (n = 10) have a CD4+CD25+CD26-CD127- phenotype.

Given that CD25, unlike CD4, is a marker that provides continuous expression, the definition of the sub-population expressing the highest levels of CD25 is not free from arbitrariness and inconsistency from experiment to experiment. Further, and as shown in Figure 2, CD4+ cells that express low levels of CD25 (and even CD25- cells) also contain CD4+FoxP3+ cells; in other words, nTregs. Therefore, an additional marker is needed to separate within CD25+ cells, those which are true nTreg.cells from those which are not.

A good marker of nTreg is FoxP3, although it is an intracellular protein. In Figure 3 (peripheral blood mononuclear cells, PBMCs) it is shown how about 6.6% of CD4+ T lymphocytes express this transcription factor, and are therefore nTreg cells; however, the cell activation process using phytohemagglutinin (PHA), which simulates an inflammatory response, increases this percentage of nTreg cells up to a "false" 16.6%, as a result of the appearance of T cells that express transient intermediate levels of FoxP3, but which in reality are not "true" nTreg cells, but rather activated effector T cells. Conversely, when said percentage of nTreg cells is estimated according to FoxP3 and CD26 simultaneously, the results are more reliable (8.8% non-activated vs. 13% activated), due to the fact that activated effector T cells have the FoxP3+CD26+ phenotype, whereas nTreg cells are FoxP3+CD26-. The same results were obtained when the cultures of cells activated with PHA were additionally stimulated with a pro - inflammatory cytokine and present in the TH1: IL-12 responses.

Figure 4 shows how CD26 allows nTreg CD4+FoxP3+ cells to be distinguished within the CD4+CD25+ cell population, which makes CD26 a useful additional marker to CD25. In the first part of figure 4 (non - activated, day 0) it can be observed how nTreg cells selected according to the CD4 and CD25 markers (R6) present 93% of FoxP3+ cells, while nTreg cells selected using markers CD4, CD25 and CD26 (R3) present an even greater wealth (95%) of FoxP3+ cells. More importantly, however, is the fact that within the CD4+CD25+ nTreg cells (R6) there is a noticeable proportion of cells with low FoxP3 levels (in other words, effector T cells), something that is practically nonexistent in CD4+CD25+CD26- cells (R3). In this same sense, the second part of Figure 4 (activated PBMCs, PHA 5 days) shows once again how nTreg cells selected exclusively according to the CD4 and CD25 markers (R6) present a 97.8% wealth of FoxP3+ cells, a practically identical proportion (98.1% FoxP3+) to the nTreg cells selected using the CD4, CD25 and CD26 markers (R3). Nonetheless, the same figure also shows the persistence of a significant proportion of cells having low levels of FoxP3 (effector T cells) within the CD4+CD25+ nTreg cells (R6), a sub-population that is clearly reduced within the CD4+CD25+CD26- (R3; Figure 4, part A and B) or CD4+CD25+CD49d-(not shown) nTreg cells. However, unlike this last marker, CD49d, CD26 presents more notable differences of expression on comparing nTreg lymphocytes (FoxP3 +) and effector lymphocytes (FoxP3weak/-) (Figure 5, part A), and also appear to be a better criterion of elimination of IFNγ producer cells within the CD4 + CD25 + nTregs (Figure 5, part B).

In conclusion, the data presented in Figures 3, 4 and 5 make it possible to affirm that the CD4+CD25+CD26- criterion in cell purification protocols would provide access to highly pure populations of nTreg cells and free of effector T cells producers of IFNγ, without being necessary to use the highly subjective CD25^{strong} phenotype, thereby allowing recovery of ∼ 60 - 70% of the initial nTreg cells (a certain percentage will be lost due to the existence of CD4+FoxP3+ cells that escape the selection process through being either CD25⁻ or CD26*^{weak}*).

## Claims

1. Method for the isolation of natural regulatory T cells (nTreg) from an isolated biological sample, comprising the following stages:
a) treating said biological sample with an anti-CD25 antibody and with an anti-CD26 antibody, and
b) separating the nTreg cells.

2. Method according to claim 1, wherein in stage (a) the biological sample is additionally treated with an anti-CD127 antibody and/or with an anti-CD49d antibody.

3. Method according to claim 1, wherein in stage (b) CD25- and CD26+ cells are removed from the biological sample.

4. Method according to claim 2, wherein in stage (b) the following cells are removed:
- CD127 + when in stage (a) the biological sample is treated with an anti-CD127 antibody, and/or
- CD49d + when in stage (a) the biological sample is treated with an anti-CD49d antibody.

5. Method according to any of claims 1 to 4, wherein in stage (b) non CD4+ T cells are removed from the biological sample.

6. Method according to claim 5, wherein in stage (a) the biological sample is additionally treated with an anti-CD4 antibody.

7. Method according to any of claims 1 to 4, wherein in stage (a) the biological sample is additionally treated with at least one antibody that recognises a molecule present in non CD4+ T cells.

8. Method according to claim 7, wherein in stage (a) the biological sample is treated with at least one antibody that recognises a molecule present in non CD4+ T cells selected from the list comprising: anti-CD8 antibody, anti-CD11b/Mac1 antibody, anti-CD14 antibody, anti-CD16 antibody, anti-CD19 antibody, anti-CD36 antibody, anti-CD41a antibody, anti-CD56 antibody, anti-CD123 antibody, anti-CD235a antibody and anti-γδTCR antibody, or any combination thereof.

9. Method according to any of claims 1 to 8, wherein in stage (a) the biological sample is treated with at least one anti-CD45RO antibody or one anti-CD45RA antibody.

10. Method according to any of claims 1 to 9, wherein at least one of the antibodies used in stage (a) is marked.

11. Method according to claim 10, wherein at least one of the antibodies used in stage (a) is marked with a label selected from the list comprising: a radioisotope, a fluorescent or luminescent marker, an antibody, an antibody fragment, an affinity label, an enzyme and an enzyme substrate.

12. Method according to any of claims 1 to 11, wherein at least one of the antibodies used in stage (a) is immobilised.

13. Method according to claim 12, wherein at least one of the antibodies used in stage (a) is immobilised in a support selected from the list comprising: a nylon matrix, a plastic support or beads.

14. Method according to any of claims 1 to 13, wherein stage (b) is carried out by means of at least one method selected from the list comprising: centrifugation, *fluorescence activated cell sorting*/*FACS, cell elutriation,* magnetic cell separation, column-based immunological separation, cell adhesion, complement-mediated lysis or flow cytometry, or any combination thereof.

15. Method according to any of claims 1 to 14, wherein the biological sample is selected from the list comprising: spleen, thymus, lymph gland, bone marrow, Peyer's patch, tonsil, synovial fluid, lymph, cerebrospinal fluid, urine, pleural fluid, pericardial fluid, peritoneal fluid, amniotic fluid, discharge, blood, leukocyte concentrate, peripheral blood mononuclear cells, peripheral blood lymphocytes or cell lines.

16. Method according to any of claims 1 to 15 wherein the biological sample is taken from an individual having an autoimmune disease, an allergic disease, an inflammatory disease, an infectious disease or a parasitic disease.

17. Kit for carrying out the method according to any of claims 1 to 16 comprising one anti-CD25 antibody and one anti-CD26 antibody.

18. Kit according to claim 17, additionally comprising one anti-CD127 antibody, and/or one anti-CD49d antibody.

19. Kit according to any of claims 17 or 18, additionally comprising one anti-CD4 antibody.

20. Kit according to any of claims 17 to 19, additionally comprising at least one antibody that recognises a molecule present in non CD4+ T cells.

21. Kit according to claim 20, wherein the antibody that recognises a molecule present in non CD4+ T cells is selected from the list comprising: anti-CD8 antibody, anti-CD11b/Mac1 antibody, anti-CD14 antibody, anti-CD16 antibody, anti-CD19 antibody, anti-CD36 antibody, anti-CD41a antibody, anti-CD56 antibody, anti-CD123 antibody, anti-CD235a antibody or anti-γδTCR antibody.

22. Kit according to any of claims 17 to 21, additionally comprising an anti-CD45RO antibody and/or an anti-CD45RA antibody.

23. Kit according to any of claims 17 to 22, wherein at least one of said antibodies is marked.

24. Kit according to claim 23, wherein at least one of the antibodies is marked with a label selected from the list comprising: a radioisotope, a fluorescent or luminescent marker, an antibody, an antibody fragment, an affinity label, an enzyme and an enzyme substrate.

25. Kit according to any of claims 17 to 24, wherein at least one of the antibodies is immobilised.

26. Kit according to claim 25, wherein at least one of the antibodies is immobilised in a support selected from the list comprising: a nylon matrix, a plastic support or beads.

27. Kit according to any of claims 17 to 26, additionally comprising at least one element necessary for the separation of cells by means of a method selected from the list comprising: centrifugation, fluorescence activated cell sorting/FACS, magnetic cell separation, column-based immunological separation, cell adhesion or complement-mediated lysis.

28. Kit according to any of claims 17 to 27, additionally comprising at least one element selected from the list comprising: columns, column supports, fraction collection tubes, magnets, meshes for removing cell aggregates, solutions containing complement factors, isotonic washes, cell enrichment/separation solutions, superparamagnetic beads, plastic plates or supports with antibodies or other bound molecules, components that allow dead cells to be removed before the purification process, solutions for the exclusion of dead cells during evaluation by flow cytometry of the purity of the nTregs once purified, antibodies bound to fluorochromes to evaluate by flow cytometry the purity of the nTregs once purified or cell permeabilisation solutions to evaluate by flow cytometry the purity of the nTregs once purified.

29. Use of the kit according to any of claims 17 to 28 for the isolation of nTreg cells from an isolated biological sample.

30. Use of the kit according to claim 29, wherein the biological sample is taken from an individual having an autoimmune disease, an allergic disease, an inflammatory disease, an infectious disease or a parasitic disease.

31. Method for the identification of nTreg cells in an isolated biological sample, comprising the following stages:
a) detecting in the biological sample the expression product of the *CD25* gene and of the *CD26* gene,
b) comparing the amount of the gene expression product detected in stage (a) with a reference amount, and
c) assigning the comparison made in stage (b) to the identification of nTreg cells, when the amount detected of the expression product of the *CD26* gene is significantly lower than the reference amount for the *CD26* gene, and when the amount detected of the expression product of the *CD25* gene is significantly higher than the reference amount for the *CD25* gene.

32. Method for the identification of nTreg cells in an isolated biological sample according to claim 31, wherein in the biological sample of stage (a) further detects the expression product of the *FoxP3, CD127* or *CD49d* gene or any combination thereof and in stage (c) the comparison made in stage (b) is assigned to the identification of nTreg cells, when also,
- the amount detected of the expression product of the *FoxP3* gene is significantly higher than the reference amount for the *FoxP3* gene, or
- the amount detected of the expression product of the *CD127* gene is significantly lower than the reference amount for the *CD127* gene, or
- the amount detected of the expression product of the *CD49d* gene is significantly lower than the reference amount for the *CD49d* gene or any combination thereof.

33. Method according to claim 32, wherein in stage (a) the expression of genes *CD25, CD26* and *FoxP3* is detected, and in stage (c) the comparison made in stage (b) is assigned to the identification of nTreg cells when the amounts of product defined for *CD25, CD26* and *FoxP3* are detected..

34. Method according to claim 32, wherein in stage (a) the expression of genes *CD25* and *CD26* is detected and also *CD127* or *CD49d,* and in stage (c) the comparison made in stage (b) is assigned to the identification of nTreg cells when the amounts of product defined for *CD25* and *CD26* and also for *CD127* or *CD49d* are detected.

35. Method according to any of claims 31 to 34 wherein in stage (a) the amount of the expression product of the CD4 gene is also detected, and in stage (c) the comparison made in stage (b) is assigned to the identification of nTreg cells, when also the amount detected of the expression product of the *CD4* gene is significantly higher than the reference amount for the *CD4* gene.

36. Method according to any of claims 31 to 35 wherein in stage (a) is also detected the amount of the expression product of at least one of the genes selected from the list comprising: *TCRαβ, CD3, CD5, CD11a*/*LFA-1, CD27, CD28, CD30, CD31, CD38, CD39, CD44, CD45RA, CD45RB, CD45RO, CD45RC, CD54*/*ICAM-1, CD57, CD58, CD62L*/*L-selectin, CD62P*/*P-selectin, CD71, CD73, CD83, CD80, CD86, CD95*/*Fas*/*APO-1, CD101, CD103, CD122*/*IL-2Rβ, CD132, CD134*/*OX-40, CD137*/*4-1BB, CD152*/*CTLA-4, CD154*/*CD40L, CD223*/*LAG-3, PD-1, PD-L1, GITR, IL-10, TGFβ, galectin-1, Neuropilin*/*NRP, Neopterin, TNFR2, TGFβR1, G-protein-coupled receptor B3*/*GPR83, HLA-DR, ICOS, GARP, FR4, TLR4, TLR5, TLR8, CRTH2, CCR7, CCR4, CCR8, CCR5, CXCR4, CXCR5, CLA, granzyme A or granzyme B.*

37. Method according to any of claims 31 to 36, wherein the expression product of the genes detected in stage (a) is mRNA.

38. Method according to any of claims 31 to 37, wherein the expression product of the genes detected in stage (a) is protein.

39. Method according to any of claims 31 to 38, wherein the biological sample is selected from the list comprising: spleen, thymus, lymph node, bone marrow, Peyer's patch, tonsil, synovial fluid, lymph, cerebrospinal fluid, urine, pleural fluid, pericardial fluid, peritoneal fluid, amniotic fluid, discharge, blood, leukocyte concentrate, peripheral blood mononuclear cells, peripheral blood lymphocytes or cell lines.

40. Method according to any of claims 31 to 39 wherein the biological sample is taken from an individual having an autoimmune disease, an allergic disease, an inflammatory disease, an infectious disease or a parasitic disease.

41. Kit for carrying out the method according to any of claims 31 to 40 comprising the elements necessary for detecting in a biological sample the expression product of the CD25 gene and of the CD26 gene, wherein said elements are selected from:
- a probe, a primer or an antibody allowing detection of the expression product of the CD25 gene and of the CD26 gene respectively, or
- a reagent allowing detection of the dipeptidyl peptidase IV activity of the CD26 protein, or
- any combination thereof.

42. Kit according to claim 41, additionally comprising the elements necessary for detecting in a biological sample the expression product of at least one of the genes selected from the list comprising: *FoxP3, CD127 or CD49d,* or any combination thereof, wherein said elements are selected from a probe, a primer or an antibody allowing detection, respectively, of the expression product of at least one gene selected from the list comprising: *FoxP3, CD127 or CD49d,* or any combination thereof.

43. Kit according to claim 42, comprising the elements necessary for detecting in a biological sample the expression product of genes *CD25, CD26* and *FoxP3* wherein said elements are selected from a probe, a primer or an antibody allowing detection, respectively, of the expression product of genes *CD25, CD26* and *FoxP3,* or any combination thereof.

44. Kit according to claim 42, comprising the elements necessary for detecting in a biological sample the expression product of genes *CD25* and *CD26,* and also, of genes *CD127* and/or *CD49d,* wherein said elements are selected from a probe, a primer or an antibody allowing detection, respectively, of the expression product of genes *CD25* and *CD26,* and also of genes *CD127* and/or *CD49d,* or any combination thereof.

45. Kit according to any of claims 41 to 44 additionally comprising a probe, a primer or an antibody allowing detection of the expression product of the *CD4* gene.

46. Kit according to any of claims 41 to 45 additionally comprising a probe, a primer or an antibody allowing detection of the expression product of at least one gene selected from the list comprising: *TCRαβ, CD3, CD5, CD11a*/*LFA-1, CD27, CD28, CD30, CD31, CD38, CD39, CD44, CD45RA, CD45RB, CD45RO, CD45RC, CD54*/*ICAM-1, CD57, CD58, CD62L*/*L-selectin, CD62P*/*P-selectin, CD71, CD73, CD83, CD80, CD86, CD95*/*Fas*/*APO-1, CD101, CD103, CD122*/*IL-2RO, CD132, CD134*/*OX-40, CD137*/*4-1BB, CD152*/*CTLA-4, CD154*/*CD40L, CD223*/*LAG-3, PD-1, PD-L1, GITR, IL-10, TGFβ, galectin-1, Neuropilin*/*NRP, Neopterin, TNFR2, TGFβR1, G-protein-coupled receptor 83*/*GPR83, HLA-DR, ICOS, GARP, FR4, TLR4, TLR5, TLR8, CRTH2, CCR7, CCR4, CCR8, CCR5, CXCR4, CXCR5, CLA, granzyme A or granzyme B.*

47. Kit according to any of claims 41 to 46, wherein at least one of the antibodies is marked.

48. Kit according to claim 47, wherein at least one of the antibodies is marked with a label selected from the list comprising: a radioisotope, a fluorescent or luminescent marker, an antibody, an antibody fragment, an affinity label, an enzyme or an enzyme substrate.

49. Kit according to any of claims 41 to 48, wherein at least one of the antibodies is immobilised.

50. Kit according to claim 49, wherein at least one of the antibodies is immobilised in a support selected from the list comprising: a nylon matrix, a plastic support or beads.

51. Kit according to any of claims 41 to 50, additionally comprising at least one element selected from the list comprising positive and/or negative controls, buffers, agents for preventing contamination, protein degradation inhibitors, polymerases, nucleotides, supports, containers or instructions.

52. Use of the kit according to any of claims 41 to 51 for the identification of nTreg cells from an isolated biological sample.

53. Use of the kit according to claim 52, wherein the biological sample is taken from an individual having an autoimmune disease, an allergic disease, an inflammatory disease, an infectious disease, or a parasitic disease.

54. Method for the purification of nTreg cells comprising:
a. treating a population of CD25+ cells with an anti-CD26 antibody, and
b. selecting the CD26- cells from the previous cell population.

55. Method according to claim 54, wherein stage (a) further comprises treating the cell population with an anti-CD127 antibody and/or with an anti-CD49d antibody, and in stage (b) selecting CD127- and/or CD49d- cells.
